# EUROPEAN PATENT APPLICATION

(11) **EP 2 977 462 A1**
(43) Date of publication of application: **27.01.2016**
(21) Application number: 14306207.3
(22) Date of filing: 25.07.2014
(51) Int. Cl.: C12P 23/00, C12N 15/63, C12N 15/74, C12P 5/00, C07K 19/00

(54) **In vivo production of a recombinant carotenoid-protein complex**

(71) Applicant: Phycosource, 95000 Cergy (FR); COMMISSARIAT A L'ENERGIE ATOMIQUE ET AUX ENERGIES ALTERNATIVES, 75015 Paris (FR); CENTRE NATIONAL DE LA RECHERCHE SCIENTIFIQUE -CNRS-, 75016 Paris (FR)
(72) Inventor: Bourcier de Carbon, Céline, 92200 Neuilly-sur-Seine (FR); Kirilovsky, Diana, 75013 Paris (FR); Boulay, Clémence, 78124 Mareil-sur-Mauldre (FR); Duran, Bertrand-Elie, 93500 Pantin (FR); Phulpin, Alexandre, 95160 Montmorency (FR); Wilson, Adjélé, 93600 Aulnay sous Bois (FR)
(74) Representative: Regimbeau

(57) **Abstract**

The present invention relates to a method for producing a carotenoid-protein complex *in vivo* comprising the steps of transforming of prokaryote cell with a plasmid containing genes involved in carotenoid synthesis and with a plasmid containing an apo-carotenoprotein gens; culturing said porokaryote cells such as to induce sequential genes expression, isolating and purifying the carotenoid-protein complex.

SEQUENCE LISTING

<110>  PHYCOSOURCE
       COMMISSARIAT A L'ENERGIE ATOMIQUE ET AUX ENERGIES
       ALTERNATIVES
       CENTRE NATIONAL DE LA RECHERCHE SCIENTIFIQUE (CNRS)

<120>  IN VIVO PRODUCTION OF A RECOMBINANT CAROTENOID-PROTEIN COMPLEX

<130>  71302D33757

<160>  69

<170>  PatentIn version 3.5

<210>  1
<211>  954
<212>  DNA
<213>  Synechocystis PCC 6803

<220>
<221>  misc_feature
<223>  OCP

<400>  1
atgccattca ccattgactc tgcccgcgga attttcccta acaccctagc ggccgacgtt      60

gtacccgcta ccatcgcccg ttttagccaa ctcaatgccg aagatcaatt agctctgatt     120

tggtttgctt acctagaaat gggtaaaacc cttacgatcg ccgcccctgg ggccgccagc     180

atgcaactgg ccgaaaatgc cctcaaggaa attcaagcca tggggcccct ccaacaaacc     240

caggccatgt gtgacttggc caaccgagct gacactcccc tgtgccgcac ctacgccagt     300

tggtccccca acattaaact tggcttctgg taccgtttag gcgaactgat ggagcagggc     360

tttgtcgccc ccattcccgc tggttaccaa ctttctgcca atgccaatgc agtgctagct     420

acgatccaag gcctagaatc cggtcaacaa attaccgtat tgcgtaatgc cgtggtggac     480

atgggcttca ccgctggtaa ggacggcaaa cgcatcgctg agcccgtagt tcctccccaa     540

gataccgcca gccgcaccaa agtttccatt gaaggggtga ccaatgccac tgtcctcaac     600

tacatggaca acctcaatgc caatgacttc gacaccttaa tcgaattgtt cacctccgac     660

ggtgccctcc aaccgccctt ccaacggccc attgtcggta aagaaaatgt gctccgcttt     720

ttccgggaag agtgccaaaa cctgaaattg atcccggaac ggggggttac tgaacccgct     780

gaagatggtt tcacccaaat taaagttacc ggtaaggtgc aaaccccttg gtttggtggc     840

aacgtgggca tgaatatcgc ctggcgcttt ctcctcaacc ccgagggcaa aattttcttt     900

gtggcgatcg acctccttgc ttcccccaaa gaattactca actttgctcg ctag           954

<210>  2
<211>  960
<212>  DNA
<213>  Arthrospira PCC 7145

<220>
<221>  misc_feature
<223>  OCP

<400>  2
atgccattca ccattgactc ggctcgtagc atttttccag aaaccctagc ggctgacgta      60

gttccggcga cgatcgctcg gttcaagcag cttagtgccg aagatcaact ggctctcatt     120

tggtttgcgt atctggaaat gggtaaaacc atcaccattg ctgctcctgg ggctgcgaat     180

atgcagtttg ctgaaaacac cctacaggaa attcggcaga tgacccccct acagcaaacc     240

caggctatgt gtgacctggc gaaccggaca gatactccca tttgtcgtac ctatgctagt     300

tggtctccta atatcaagct gggtttctgg tatgaactag ggcgtttcat ggatcaagga     360

cttgttgcgc ccattcccga aggctacaaa ctctcagcta acgccaacgc tatcctagtg     420

actattcaag gtattgatcc aggtcaacaa atcacggtgc tgcgtaattg tgttgttgat     480

atggggttcg ataccagcaa attaggcagc tatcaacggg tagcagaacc tgttgtacct     540

cctcaagaga tgtcccaacg gactaaagtt caaatcgaag gtgtgaccaa ttccacagtc     600

ctgcaataca tggacaactt gaatgctaac gattttgata acttaattag tctgtttgca     660

gaagatggag ctttacaacc tcctttccaa aaaccaattg ttggtaaaga gaatgtgctg     720

cggttcttcc gggaagaatg ccagaacctg aagttaatcc cagaacgagg tgtttctgaa     780

ccgacggaag atggctatac tcagattaag gttactggta aagtgcaaac tccctggttt     840

ggtggtaatg tggggatgaa tattgcttgg cgcttcttgc tgaacccaga aaataaggtt     900

ttctttgtgg cgatcgattt gttagcatct cccaaagagt tgttgaactt ggtgcgctag     960


<210>  3
<211>  1075
<212>  DNA
<213>  Anabaena PCC 7120


<220>
<221>  misc_feature
<223>  OCP

<400>  3
atggcaatta ctatcgattc cgcccgtcgt atcttcccta acacattaca ggctgatgca      60

gtgccagcgc tgactgcacg attcaaccaa ctcagtgcag aagatcaact ggcatggaca     120

tggttcgctt tcttagagat gggcaaaact attactgttg cagcgcctgg tgcagctagt     180

atgcagtttg cggaaggtat cctcaagcaa atcaaggaaa tgacctttga ggaacaaact     240

caggttatgt gcgacctggc aaaccacaca gatacgccca tttgccgcac ctatgctact     300

tggtcgccta atatcaagtt aggtttttgg aatcaactgg gagaatggat ggagcaaggt     360

gccgttgcgc caattcctgc tggctatcag ctttcagcca atgctaatgc agtattggaa 420

accctgaaaa gtctggatca aggacaacag attaccgtcc tccgcagttc cgttgtagat 480

atgggatttg atgcagctaa actagacggc tacacaagag ttgctgaacc attagttgca 540

cctaaagata tttcccagcg tgttcaagtc accattgagg gaattaataa ttccactgtg 600

ttgaattaca tgaacaactt gaacgccaat gactttgatg aactgatcaa gttgttcgtg 660

gaggatggag ctttgcagcc tcccttccaa agaccaatcg ttggtaagga tgcaattctg 720

cggttcttcc gtgaagaatg ccagaacctc aacttgcttc cagagcgcgg tgtggctgaa 780

cccgcagacg atggctatac tcaggtgaaa gtgacaggta aagttcagac cccttggttt 840

ggtgcagcag tagggatgaa catggcttgg cgcttcttgc tcaaccccca aggtaaaatc 900

ttcttcgtag ctattgactt gttagcctct cccaaagaac ttttgaactt ggttcgctaa 960

aggaaaataa caaaacaact aaatagctga ttatcgcggt gagcgctctc ttaacgaagg 1020

gcgctcaatt attctgagca aacgcaatta attaatacaa gtttaagatt gagtc 1075

<210> 4
<211> 492
<212> DNA
<213> Anabaena PCC 7120

<220>
<221> misc_feature
<223> RCP

<400> 4
atgactttta cacaaacaaa cgatcccact attcgtaaat atgtccaatc atggcagaaa 60

ttggatgtag acgaacaatt ggctttgttt tggtttatct acaaagaaat gggtggtgca 120

attacaccag cagcccctgg tgctagcact gtttccccag caattgccga aggtttattt 180

aatcaagtca aagaattgaa tcatgaacag caattgcaac tacaacgcga cttaattcgg 240

cgtgtagata accaactcag ccgcgaatac ggctctctgg gtgataccac taagctactg 300

ttttggtatc ttttatctca aggaatggat aatgcaacca tagttccttt tcctgccgat 360

tataaactgt cttcagaatc ccaagaactg ttggaaggaa ttaaaggctt aggttttgaa 420

caacagatta ctcttttccg tgattacgtt tccccaatgg gtgctgaagc aaaagcgggt 480

gcggaaattt ag 492

<210> 5
<211> 1629
<212> DNA
<213> Synechocystis PCC 6803

<220>
<221> misc_feature

<223>  CrtO(beta-carotene ketolase)

<400>  5
atgatcacca ccgatgttgt cattattggg gcggggcaca atggcttagt ctgtgcagcc      60

tatttgctcc aacggggctt gggggtgacg ttactagaaa agcgggaagt accagggggg     120

gcggccacca cagaagctct catgccggag ctatcccccc agtttcgctt taaccgctgt     180

gccattgacc acgaatttat ctttctgggg ccggtgttgc aggagctaaa tttagcccag     240

tatggtttgg aatatttatt ttgtgacccc agtgtttttt gtccggggct ggatggccaa     300

gcttttatga gctaccgttc cctagaaaaa acctgtgccc acattgccac ctatagcccc     360

cgagatgcgg aaaaatatcg gcaatttgtc aattattgga cggatttgct caacgctgtc     420

cagcctgctt ttaatgctcc gccccaggct ttactagatt tagccctgaa ctatggttgg     480

gaaaacttaa aatccgtgct ggcgatcgcc gggtcgaaaa ccaaggcgtt ggattttatc     540

cgcactatga tcggctcccc ggaagatgtg ctcaatgaat ggttcgacag cgaacgggtt     600

aaagctcctt tagctagact atgttcggaa attggcgctc ccccatccca aaagggtagt     660

agctccggca tgatgatggt ggccatgcgg catttggagg gaattgccag accaaaagga     720

ggcactggag ccctcacaga agccttggtg aagttagtgc aagcccaagg gggaaaaatc     780

ctcactgacc aaaccgtcaa acgggtattg gtggaaaaca accaggcgat cggggtggag     840

gtagctaacg gagaacagta ccgggccaaa aaaggcgtga tttctaacat cgatgcccgc     900

cgtttatttt tgcaattggt ggaaccgggg gccctagcca aggtgaatca aaacctaggg     960

gaacgactgg aacggcgcac tgtgaacaat aacgaagcca ttttaaaaat cgattgtgcc    1020

ctctccggtt taccccactt cactgccatg gccgggccgg aggatctaac gggaactatt    1080

ttgattgccg actcggtacg ccatgtcgag gaagcccacg ccctcattgc cttggggcaa    1140

attcccgatg ctaatccgtc tttatatttg gatattccca ctgtattgga ccccaccatg    1200

gccccccctg ggcagcacac cctctggatc gaattttttg ccccctaccg catcgccggg    1260

ttggaaggga cagggttaat gggcacaggt tggaccgatg agttaaagga aaaagtggcg    1320

gatcgggtga ttgataaatt aacggactat gcccctaacc taaaatctct gatcattggt    1380

cgccgagtgg aaagtcccgc cgaactggcc caacggctgg gaagttacaa cggcaatgtc    1440

tatcatctgg atatgagttt ggaccaaatg atgttcctcc ggcctctacc ggaaattgcc    1500

aactaccaaa cccccatcaa aaatctttac ttaacagggg cgggtaccca tcccggtggc    1560

tccatatcag gtatgcccgg tagaaattgc gctcgggtct ttttaaaaca acaacgtcgt    1620

ttttggtaa                                                            1629

<210>  6
<211>  939
<212>  DNA

<213>  Synechocystis PCC 6803

<220>
<221>  misc_feature
<223>  CrtR(beta-carotene ketolase)

<400>  6
ctagggcttg tcagatgatt gagaattatt atggccgtga aaacgaatac caaggaaaac      60

atcatagagg aagctccaga aatttttccc ttccagtaat cccagggatt gatcacaacc     120

cttctcatcc aaaatgggct tggtgatgtg ataggtgttt tggtactgat accaaggaat     180

agaaggccaa aggtggtgga tcaggtggta attttgcccg aaaatgagcc aatttaaaat     240

ggggctggga taaaccctgg catttttcca acggttgcgt tcttggaaag gtcgatgggg     300

caggtaatca aaaaacagtc ccagggcaat gccaacaatt aaagcaggca caaaccagta     360

attcatcaca aagccgataa agccgtaatg aatgccgaga aaaacgatcg tgaacaacac     420

aagccgactt aagaaccact ctagtagctc atatttgcgc cataaccgcc gtttaaagaa     480

aaagatctca tggtagaaga accgggcggc aatgaggaag aggggaccgc cggtggaaac     540

aaaatggtct gggtcatttt cagggtcatt gacgttggcg tggtgttgga gatgaacccg     600

ggtaaagacg ggaaaagcaa agcccaacat taaggcacta ccgtggccaa gcactgcatt     660

aataatggtg ttccgatggg ccgcattgtg ggatgcatca tggatcactg tgcctgacag     720

gtgcagcgcc agcaccgaac aactaaaaca aagccagtgg ggcagccccc attgccagta     780

gccaaaaacg ttagcgagca ttaagagaat agctatcccg aacatggcca cgttgggatt     840

gaagccgccg tccgcctgta aaaactcttt tgggacagct tgggaaacgg tttgcagcgg     900

ttgggtcgcc tgcattacta tgacggactc ctggcacac                            939

<210>  7
<211>  777
<212>  DNA
<213>  Anabaena PCC 7120

<220>
<221>  misc_feature
<223>  CrtW(beta-carotene ketolase)

<400>  7
atggttcagt gtcaaccatc atctctgcat tcagaaaaac tggtgttatt gtcatcgaca      60

atcagagatg ataaaaatat taataagggt atatttattg cctgctttat cttattttta     120

tgggcaatta gtttaatctt attactctca atagatacat ccataattca taagagctta     180

ttaggtatag ccatgctttg gcagaccttc ttatatacag gtttatttat tactgctcat     240

gatgccatgc acggcgtagt ttatcccaaa aatcccagaa taaataattt tataggtaag     300

ctcactctaa tcttgtatgg actactccct tataaagatt tattgaaaaa acattggtta     360

caccacggac atcctggtac tgatttagac cctgattatt acaatggtca tccccaaaac      420

ttctttcttt ggtatctaca ttttatgaag tcttattggc gatggacgca aattttcgga      480

ttagtgatga tttttcatgg acttaaaaat ctggtgcata taccagaaaa taatttaatt      540

atattttgga tgataccttc tattttaagt tcagtacaac tattttattt tggtacattt      600

ttgcctcata aaaagctaga aggtggttat actaaccccc attgtgcgcg cagtatccca      660

ttacctcttt tttggtcttt tgttacttgt tatcacttcg gctaccacaa ggaacatcac      720

gaataccctc aacttccttg gtggaaatta cctgaagctc acaaaatatc tttataa         777


<210>  8
<211>  42
<212>  DNA
<213>  Artificial

<220>
<223>  NpCDFduet

<400>  8
atgggcagca gccatcacca tcatcaccac agccaggatc cg                          42


<210>  9
<211>  48
<212>  DNA
<213>  Artificial

<220>
<223>  NC15

<400>  9
atgggcagca gcaatcaggc taatcaggtg actctaaatc cacaagtt                    48


<210>  10
<211>  33
<212>  DNA
<213>  Artificial

<220>
<223>  NC11

<400>  10
atgggcagca gcaatcaggc taatcaggtg act                                    33


<210>  11
<211>  27
<212>  DNA
<213>  Artificial

<220>
<223>  NC9

<400>  11
atgggcagca gcaatcaggc taatcag                                           27

<210>  12
<211>  21
<212>  DNA
<213>  Artificial

<220>
<223>  NC7

<400>  12
atgggcagca gcaatcaggc t                                                21


<210>  13
<211>  18
<212>  DNA
<213>  Artificial

<220>
<223>  NC6

<400>  13
atgggcagca gcaagagg                                                    18


<210>  14
<211>  12
<212>  DNA
<213>  Artificial

<220>
<223>  NC4

<400>  14
atgggcagca gc                                                          12


<210>  15
<211>  48
<212>  DNA
<213>  Artificial

<220>
<223>  MIX15

<400>  15
atgggcagca gccgactaga caatccagaa aaaactgaca ttgaacca                   48


<210>  16
<211>  27
<212>  DNA
<213>  Artificial

<220>
<223>  C9

<400>  16
atgggcagca gcaaaaagag aagggct                                          27


<210>  17
<211>  30

<212>  DNA
<213>  Artificial

<220>
<223>  HisTagNter

<400>  17
atgggcagca gccatcacca tcatcaccac                                        30


<210>  18
<211>  18
<212>  DNA
<213>  Artificial

<220>
<223>  HisTagCter

<400>  18
caccaccacc accaccac                                                     18


<210>  19
<211>  30
<212>  DNA
<213>  Artificial

<220>
<223>  Primer CrtO F pBADgIII-CrtO

<400>  19
aacagaagat ctatcaccac cgatgttgtc                                        30


<210>  20
<211>  30
<212>  DNA
<213>  Artificial

<220>
<223>  Primer CrtO R pBADgIII-CrtO

<400>  20
tctgttgaat tcgcttcact tgccatacac                                        30


<210>  21
<211>  48
<212>  DNA
<213>  Artificial

<220>
<223>  Primer pBAD-CrtO

<400>  21
caggaggaat taaccatgat caccaccgat gttgtcatta ttggggcg                    48


<210>  22
<211>  48
<212>  DNA
<213>  Artificial

<220>
<223>  Primer pBAD-CrtO

<400>  22
catggttaat tcctcctgtt agcccaaaaa acgggtatgg agaaacag                    48


<210>  23
<211>  48
<212>  DNA
<213>  Artificial

<220>
<223>  Primer pBAD/gIIIAmut F pBAD

<400>  23
caggaggaat taaccatgac catggagctc gagatctgca gctggtac                    48


<210>  24
<211>  48
<212>  DNA
<213>  Artificial

<220>
<223>  Primer pBAD/gIIIAmut R pBAD

<400>  24
catggttaat tcctcctgtt agcccaaaaa acgggtatgg agaaacag                    48


<210>  25
<211>  32
<212>  DNA
<213>  Artificial

<220>
<223>  Primer CrtR F pBAD-crtR

<400>  25
aacagaccat gggctgccag gagtccgtca ta                                     32


<210>  26
<211>  30
<212>  DNA
<213>  Artificial

<220>
<223>  Primer CrtR R pBAD-crtR

<400>  26
tctgttctcg agcaattact tacccgattt                                        30


<210>  27
<211>  31
<212>  DNA
<213>  Artificial

<220>
<223>  Primer CrtW F pBAD-crtW

<400>  27
gatataccat ggttcagtgt caaccatcat c                                    31


<210>  28
<211>  28
<212>  DNA
<213>  Artificial

<220>
<223>  Primer CrtW R pBAD-crtW

<400>  28
gcatcggaat tcagtcgtat tccagcag                                        28


<210>  29
<211>  31
<212>  DNA
<213>  Artificial

<220>
<223>  Primer OCPsynHistagNter15 F pCDF-OCPsynHisTagNter15

<400>  29
aacagagaat tctccattca ccattgactc t                                    31


<210>  30
<211>  32
<212>  DNA
<213>  Artificial

<220>
<223>  Primer OCPsynHistagNter15 R pCDF-OCPsynHisTagNter15

<400>  30
tctgttgcgg ccgctgtaac attaacttat ga                                   32


<210>  31
<211>  33
<212>  DNA
<213>  Artificial

<220>
<223>  Primer OCParthroHistagNter15 F pCDF-OCParthroHisTagNter15

<400>  31
aacagagaat tctccattca ccattgactc ggc                                  33


<210>  32
<211>  37
<212>  DNA
<213>  Artificial

<220>
<223>  Primer OCParthroHistagNter15 R pCDF-OCParthroHisTagNter15

<400>  32
tctgttgcgg ccgcttatca ttacctaatt gagcatt                              37

<210>  33
<211>  33
<212>  DNA
<213>  Artificial

<220>
<223>  Primer OCPanaHistagNter15 F pCDF-OCPanaHisTagNter15

<400>  33
aacagagaat tctgcaatta ctatcgattc cgc                                    33


<210>  34
<211>  34
<212>  DNA
<213>  Artificial

<220>
<223>  Primer OCPanaHistagNter15 R pCDF-OCPanaHisTagNter15

<400>  34
tctgttgcgg ccgcgactca atcttaaact tgta                                   34


<210>  35
<211>  33
<212>  DNA
<213>  Artificial

<220>
<223>  Primer RCPanaCter F pCDFDuet-NC2-1123HIS

<400>  35
gatataccat gggcactttt acacaaacaa acg                                    33


<210>  36
<211>  35
<212>  DNA
<213>  Artificial

<220>
<223>  Primer RCPanaCter R pCDFDuet-NC2-1123HIS

<400>  36
cattatgcgg ccgcctaatg atgatgatga tgatg                                  35


<210>  37
<211>  28
<212>  DNA
<213>  Artificial

<220>
<223>  Primer pCDF-OCPsynCter NcoI/NotI

<400>  37
caaggaaatt caagcgatgg ggcccctc                                          28


<210>  38
<211>  28

<212>  DNA
<213>  Artificial

<220>
<223>  Primer pCDF-OCPsynCter NcoI/NotI

<400>  38
gaggggcccc atcgcttgaa tttccttg                                          28


<210>  39
<211>  29
<212>  DNA
<213>  Artificial

<220>
<223>  Primer OCPsynCter F

<400>  39
aacagaccat ggcattcacc attgactct                                         29


<210>  40
<211>  32
<212>  DNA
<213>  Artificial

<220>
<223>  Primer OCPsynCter R

<400>  40
tctgttgcgg ccgctgtaac attaacttat ga                                     32


<210>  41
<211>  57
<212>  DNA
<213>  Artificial

<220>
<223>  Primer pCDF-OCPsynCterNterNC15

<400>  41
aatcaggcta atcaggtgac tctaaatcca caagttccat tcaccattga ctctgcc          57


<210>  42
<211>  60
<212>  DNA
<213>  Artificial

<220>
<223>  Primer pCDF-OCPsynCterNterNC15

<400>  42
aacttgtgga tttagagtca cctgattagc ctgattgctg ctgcccatgg tatatctcct       60


<210>  43
<211>  57
<212>  DNA
<213>  Artificial

<220>
<223>  Primer pCDF-OCPsynCterNterMIX15

<400>  43
cgactagaca atccagaaaa aactgacatt gaaccaccat tcaccattga ctctgcc          57


<210>  44
<211>  60
<212>  DNA
<213>  Artificial

<220>
<223>  Primer pCDF-OCPsynCterNterMIX15

<400>  44
tggttcaatg tcagtttttt ctggattgtc tagtcggctg ctgcccatgg tatatctcct       60


<210>  45
<211>  46
<212>  DNA
<213>  Artificial

<220>
<223>  Primer pCDF-OCPsynCterNterNC11

<400>  45
aatcaggcta atcaggtgac tccattcacc attgactctg cccgcg                      46


<210>  46
<211>  45
<212>  DNA
<213>  Artificial

<220>
<223>  Primer pCDF-OCPsynCterNterNC11

<400>  46
agtcacctga ttagcctgat tgctgctgcc catggtatat ctcct                       45


<210>  47
<211>  42
<212>  DNA
<213>  Artificial

<220>
<223>  Primer pCDF-OCPsynCterNterNC9

<400>  47
aatcaggcta atcagccatt caccattgac tctgcccgcg ga                          42


<210>  48
<211>  39
<212>  DNA
<213>  Artificial

<220>
<223>  Primer pCDF-OCPsynCterNterNC9

<400>  48
ctgattagcc tgattgctgc tgcccatggt atatctcct                              39


<210>  49
<211>  30
<212>  DNA
<213>  Artificial

<220>
<223>  Primer pCDF-OCPsynCterNterNC7

<400>  49
ccattcacca ttgactctgc ccgcggaatt                                        30


<210>  50
<211>  48
<212>  DNA
<213>  Artificial

<220>
<223>  Primer pCDF-OCPsynCterNterNC7

<400>  50
agagtcaatg gtgaatggag cctgattgct gctgcccatg gtatatct                    48


<210>  51
<211>  30
<212>  DNA
<213>  Artificial

<220>
<223>  Primer pCDF-OCPsynCterNterNC4

<400>  51
ccattcacca ttgactctgc ccgcggaatt                                        30


<210>  52
<211>  42
<212>  DNA
<213>  Artificial

<220>
<223>  Primer pCDF-OCPsynCterNterNC4

<400>  52
agagtcaatg gtgaatgggc tgctgcccat ggtatatctc ct                          42


<210>  53
<211>  42
<212>  DNA
<213>  Artificial

<220>
<223>  Primer pCDF-OCPsynCterNterC9

<400>  53
aaaaagagaa gggctccatt caccattgac tctgcccgcg ga                          42

<210>  54
<211>  39
<212>  DNA
<213>  Artificial

<220>
<223>  Primer pCDF-OCPsynCterNterC9

<400>  54
agcccttctc tttttgctgc tgcccatggt atatctcct                              39


<210>  55
<211>  33
<212>  DNA
<213>  Artificial

<220>
<223>  Primer pCDF-OCPsynNter

<400>  55
catcaccatc atcaccaccc attcaccatt gac                                    33


<210>  56
<211>  33
<212>  DNA
<213>  Artificial

<220>
<223>  Primer pCDF-OCPsynNter

<400>  56
gtggtgatga tggtgatggc tgctgcccat ggt                                    33


<210>  57
<211>  29
<212>  DNA
<213>  Artificial

<220>
<223>  Primer OCParthroCter F pCDF-OCParthroCter NcoI/NotI

<400>  57
aacagaccat ggcattcacc attgactcg                                         29


<210>  58
<211>  37
<212>  DNA
<213>  Artificial

<220>
<223>  Primer OCParthroCter R pCDF-OCParthroCter NcoI/NotI

<400>  58
tctgttgcgg ccgcttatca ttacctaatt gagcatt                                37


<210>  59
<211>  33

<212>  DNA
<213>  Artificial

<220>
<223>  Primer pCDF-OCParthroNter

<400>  59
catcaccatc atcaccaccc attcaccatt gac                                    33


<210>  60
<211>  33
<212>  DNA
<213>  Artificial

<220>
<223>  Primer pCDF-OCParthroNter

<400>  60
gtggtgatga tggtgatggc tgctgcccat ggt                                    33


<210>  61
<211>  29
<212>  DNA
<213>  Artificial

<220>
<223>  Primer OCPanaCter F pCDF-OCPanaCter NcoI/NotI

<400>  61
aacagaccat ggcaattact atcgattcc                                         29


<210>  62
<211>  34
<212>  DNA
<213>  Artificial

<220>
<223>  Primer OCPanaCter R pCDF-OCPanaCter NcoI/NotI

<400>  62
tctgttgcgg ccgcgactca atcttaaact tgta                                   34


<210>  63
<211>  36
<212>  DNA
<213>  Artificial

<220>
<223>  Primer pCDF-OCPanaNter

<400>  63
catcaccatc atcaccacgc aattactatc gattcc                                 36


<210>  64
<211>  33
<212>  DNA
<213>  Artificial

<220>
<223>  Primer pCDF-OCPanaNter

<400>  64
gtggtgatga tggtgatggc tgctgcccat ggt                                    33


<210>  65
<211>  47
<212>  DNA
<213>  Artificial

<220>
<223>  Primer pCDF-NC9-1123HIS

<400>  65
agcagcaatc aggctaatca gacttttaca caaacaaacg atcccac                     47


<210>  66
<211>  47
<212>  DNA
<213>  Artificial

<220>
<223>  Primer pCDF-NC9-1123HIS

<400>  66
ctgattagcc tgattgctgc tgcccatggt atatctcctt attaaag                     47


<210>  67
<211>  48
<212>  DNA
<213>  Artificial

<220>
<223>  Primer pCDF-NC6-1123HIS

<400>  67
ggagatatac catgggcagc agcaagagga cttttacaca aacaaacg                    48


<210>  68
<211>  32
<212>  DNA
<213>  Artificial

<220>
<223>  Primer pCDF-NC6-1123HIS

<400>  68
gcccatggta tatctcctta ttaaagttaa ac                                     32


<210>  69
<211>  528
<212>  DNA
<213>  Erwinia


<220>
<221>  misc_feature

<223>  Gene sequence of Erwinia crtz gene

<400>  69
atgttgtgga tttggaatgc cctgattgtt ctggtcactg ttatcggaat ggagataacg      60

gctgcactgg cgcacagata cattatgcat ggctggggtt ggggctggca tctgtcacat     120

catgaaccgc ataaaggctg gtttgaggtt aatgacctct atgccgtagt gttcgccgct     180

ctgtcgattt tgctcattta tctgggcagc acgggtgtct ggcccttgca gtggatcggc     240

gcaggtatga cgctctacgg cctgctctat tttattgtgc atgacggcct ggtgcatcag     300

cgctggccat tccgctatgt tccgcgccgg ggttatttac gcaggcttta tatggcgcac     360

cgcatgcatc atgcggtacg aggcaaagag ggctgtgtct cgtttggctt tctttacgcg     420

ccgccgctgt caaaactgca ggcgacgctg cgagaacgtc atggcgttaa acggggcgct      480

gccagagatc agcggagcgt ggagcgtgac gcgccacccg ggaagtaa                  528

## Description

The present invention relates to the field of recombinant proteins.

Especially, the present application relates to a method for producing *in vivo* a carotenoid-protein complex and to the carotenoid-protein complex such obtained.

The present invention further relates to a modified apo-carotenoproteine genes encoding a modified carotenoid-protein complex.

The human body is constantly exposed to external harmful factors such as ultraviolet radiation, pollution, cigarette smoke, toxic chemicals, and certain metallic ions. Many of these pollutants induce the formation of reactive oxygen species (ROS) to some extent. In addition to exogenous factors, internal factors also contribute to the formation of ROS. Metabolic reactions in all living cells are accompanied by the oxidation of nutrients while reducing oxygen to water molecules.

The formation of reactive oxygen species (ROS) and the damage caused by their potent chemical activity on their surrounding environment is ubiquitous in nearly all physiological settings. Incomplete reduction of the oxygen molecule (³O₂, the ground state of the molecule) results in the formation of ROS, including the superoxide anion (O₂^{•}-), hydroperoxyl radical (HOO•), hydroxylradical (•OH), nitric oxide (•NO), and compounds that are not free radicals but have a big reactivity such as singlet oxygen (¹O₂) and hydrogen peroxide (H₂O₂).

Physiological damage induced by ROS includes oxidation of lipids, proteins and DNA.

Due to the nocive effects of ROS, there is a big interest in the production of new antioxidant molecules having better efficiency than the antioxidants currently known in the art.

In photosynthetic organisms, carotenoids have a photoprotective role through a number of different mechanisms. By quenching chlorophyll triplet states, they prevent the energy transfer-mediated formation of singlet oxygen. Chlorophyll triplets are formed with a low but significant yield, during excitation energy transfer in light-harvesting proteins and/or after charge recombination in reaction centers. Carotenoids can additionally directly quench singlet oxygen. More recently it was shown that, in both plants and cyanobacteria, carotenoids play an essential role in regulating the amount of excitation energy reaching the reaction centers in high light environments, thus preventing damage due to overexcitation of these proteins.

For majority of applications in need of anti-oxidants, the anti-oxidant effect is expected in a water-based medium. Thus, a hydrophilic antioxidant is required. However, the most efficient antioxidant, such as carotenoids are strongly hydrophobic compounds. Current hydrophilic antioxidant, such as ascorbic acid, tocopherol or citric acid are 100 to 1000 fold less protective than common carotenoid (Yasuhiro et *al*., 2007), so that the way to use carotenoids and other lipophilic antioxidant in aqueous environment has been long time studied.

The most common way to provide such lipophilic antioxidants requires an additional step, such as performing a hydrocolloid emulsion or saponification and micronization (WO 2009022034) or derivatization with hydrophilic function. This additional step increases the cost of methods providing efficient lipophilic antioxidant in aqueous environment.

Furthermore, performing such additional step usually leads to a decrease of antioxidant efficiency (especially related to the lack of disponibility of the antioxidant in suspension) and is not suitable for all applications due to the introduction of new compounds necessary to perform the additional step.

Thus, there remains a need to provide efficient, rapid and inexpensive methods for production of a stable, water soluble and efficient anti-oxidant compound.

Carotenoids are highly hydrophobic. Immersed in the hydrophobic core of the lipid bilayer the carotenoid is solvent protected by the amphiphilic nature of the phospholipids. In photosynthetic organisms, in addition to be solubilized in the membranes, the carotenoids are mostly associated to membrane chlorophyll proteins.

However, there also exist water soluble carotenoid binding proteins. Only a small number of proteins of this family was described and characterized till now days: for ex: the cyanobacterial "Orange Carotenoid Protein" (OCP) ((Kerfeld et al., 2003)) binding 3'-hydroxiechinenone and the cyanobacterial "Red Carotenoid Protein" (RCP) ((Sedoud et al., 2014)); an Astaxanthin carotenoid protein (AstaP) isolated from an eukaryotic microalgae (Kawasaki et al., 2013) .

In human, when carotenoids are transported through the plasma, they are incorporated into lipoproteins (lipocalins). Carotenoids can also assemble with lipo(glycol) proteins. Finally, there exist a pi isoform of the glutathione-S-transferase (GSTP1) specifically binding zeaxanthin (Bhosale and Bernstein, 2005). Although there are very few well characterized examples of carotenoproteins, they are likely to be much more abundant in nature and can have important roles as protectants against ROS.

Crustacyanin, present in the lobster's carapace is one example of the family of astaxanthin or echinenone carotenoprotein family common in invertebrate marine animals (Zagalsky et al., 1990).

To produce these carotenoproteins, the scientists over-express only the apo-carotenoprotein (protein without carotenoid) in prokaryote cell such as E coli and then once the apo-protein has been isolated, the carotenoid is attached by in vitro reconstitution (examples in Giuffra et al., 1996; Bhosale and Bernstein, 2005).

Alternatively they isolate them from the original organisms, for example OCP and AstaP (Wilson et al., 2008; Kawasaki et al., 2013), or from Synechocystis mutants overexpressing these proteins, for example OCP and RCP (Sedoud et al., 2014). The inventors have tested the OCP yield obtained by others conventional methods well known in the art and the obtained results have shown that only 40 mg of OCP have been obtained from 30 liter of Synechocystis culture (1.33 mg per liter) after 3 weeks (2 weeks for growth cells and 4 days for the isolation of the protein).

Not only such standard step methods are long but also the obtained carotenoproteins in the case of reconstitution in vitro are not always completely reconstituted or functional.

Moreover, there reminds need to provide a soluble proteins (apo-carotenoproteins) able to attach the lipid soluble carotenoid with high efficiency in order to obtain very high quantity of carotenoproteins.

The purpose of the invention is to fulfill the needs of methods allowing the production of a functional carotenoid-protein complex in high quantity which makes it possible to solve in whole or part the above mentioned problems.

Unexpectedly, the inventors have demonstrated that to obtain a large quantity of functional carotenoproteins *in vivo* in the prokaryotic cells it is necessary to first inducing the expression of genes encoding the carotenoids and then, inducing the expression of genes encoding apo-carotenoprotein.

The major advantage of the present invention lies in a manner to transform prokaryotic cells to be able to synthesize the apo-carotenoproteins with an attached carotenoid in high quantities.

These results are surprising since contrary to the prior art methods carotenoid is attached to apo-carotenoprotein in host cell. In fact, this could be even more complicated with soluble carotenoid proteins since the isolated carotenoids are lipid-soluble and present only in membranes.

Thus, the invention describes a biomimetic way to obtain the full anti-oxidant effect of carotenoid in aqueous environment by using carotenoid-coupled proteins such as "natural carriers".

In one aspect the invention relate to a method for producing a carotenoid-protein complex *in vivo* comprising the steps of :
a) transforming of prokaryote cells with at least one plasmid containing the genes involved in carotenoid synthesis and with a plasmid containing a apo-carotenoprotein gene, wherein said plasmids contain different replication origins and different selective pressure;
b) culturing of transformed prokaryote cells in step a) in conditions allowing sequential gene expression,
c) isolating and purifying the protein-carotenoid complex expressed by the prokaryote cells.

Unless defined otherwise, all technical scientific terms used herein have the same meaning as commonly understood to one skilled in the art.

For convenience, the meaning of certain terms and phrases employed in the specification, examples and claims are provide.

As used herein the terms "carotenoprotein" or "carotenoid-protein complex" refer to a protein attached to carotenoid.

As used herein the term "prokaryote cell" refers to prokaryote cells have not a membrane bound nucleus, mitochondria, or any other membrane-bound organelles.

According to the present invention the prokaryote cells are selected in the group of non-photosynthetic prokaryote cells, preferably comprising *E. coli* or *Lactococcus lactis.*

According to the present invention *E. coli* strains BL21-Gold bought from Agilent Technologies (Santa Clara, CA95051-7201, USA) are used as host to construct E coli strains synthesizing carotenoids., Preferably these strains are : BL21-pßcarotene, BL21-pßcarotene, BL21echi, BL21echiBIS, BL21zea and BL21cantha (table 6 in the Examples).

As used herein the term "plasmid" or "vector" refers to a small, circular, doublestranded DNA molecule that is distinct from a cell's chromosomal DNA and that occurs in many bacterial strains.

Any one of plasmids known by the skilled in the art may be used in the present application.

Preferably the plasmids used in the present invention, their characteristics and their sources are shown in the table 4 in the Examples.

According to one embodiment of the present application the prokaryote cells are transformed with at least one plasmid containing the genes involved in carotenoid synthesis.

According to the present invention the genes encoding enzymes involved in carotenoid synthesis may be obtained from any organism selected in the group of any bacteria, any algae, any plant and any animal.

Preferably, the genes encoding carotenoids synthesis may be obtained from all algae or all eubacteria comprising bacteriochlorophyll photosynthetic bacteria, cyanobacteria and non photosynthetic eubacteria. More preferably these genes are obtained from cyanobacteria strains selected in the group of *Synechocystis sp., Anabaena sp.* and *Arthrospira sp.* or from non photosynthetic eubacteria, selected in the group of *Erwinia sp, Brevundinomonas spSD212, Paracoccus sp, and* more preferably from *Erwinia sp.*

According to the particular embodiment, the genes involved in the ß-carotene synthesis *crtE, crtB, crtI* and *crtY* are obtained from *Erwinia.*

According to another embodiment of the present invention the prokaryote cells transformed with the plasmid containing the genes involved in ß-carotene synthesis may be transformed with a second plasmid containing the genes encoding enzymes involved in the synthesis of others carotenoids.

Preferably, the prokaryote cells transformed with the plasmid containing the genes involved in ß-carotene synthesis may be transformed with a second plasmid containing the genes encoding a ß-carotene ketolase or the genes encoding a ß-carotene hydrolase.

Said genes encoding a ß-carotene ketolase or the genes encoding a ß-carotene hydrolase are involved in the synthesis of all xanthophylls (carotenoids containing at least one oxygen atom).Preferably, said genes are selected in the group comprising the genes involved in the synthesis of echinenone, canthaxanthin, zeaxanthin, astaxanthin, hydroxyechinenone, neoxanthin, violaxanthin, diadinoxanthin and fucoxanthin. More preferably, said genes are selected in the group comprising the genes involved in the synthesis of echinenone, hydroxyechinenone, canthaxanthin, zeaxanthin and astaxanthin.

According to the invention, the genes encoding ß-carotene ketolase are selected in the group comprising or consisting in crtO and crtW genes isolated from all algae or eubacteria, preferably from cyanobacteria, more preferably from cyanobacteria strains selected in the group comprising or consisting of *Synechocystis sp., Anabaena sp. or Arthrospira sp., Thermosynechococcus elongatus, Synechococcus sp,* and *Gloeobacter sp*

Still more preferably, the genes encoding a ß-carotene ketolase are *CrtO from Synechocystis* (SEQ ID NO: 5) and *CrtW* from Anabaena (SEQ ID NO: 7) which are necessary for the synthesis of echinenone and canthaxanthine respectively.

According to the invention, the genes encoding ß-carotene hydrolase are selected in the group comprising or consisting of crtZ from eubacteria (SEQ ID NO: 69) and crtR from all eubacteria, algae or cyanobacteria strains, preferably selected in the group of cyanobacterial strains *Synechocystis sp., Anabaena sp. or Arthrospira sp., Thermosynechococcus elongatus,* and *Synechococcus sp*

More preferably, the gene encoding a ß-carotene hydrolase is *CrtR* from Synechocystis (SEQ ID NO: 6) which is necessary for the synthesis of zeaxanthin.

According to the present application the sequences of primers used for cloning the genes encoding carotenoids may be synthesized by any method well known to person skilled in the art.

Preferably the sequences of primers used for cloning the genes encoding carotenoid *Crt* genes correspond to SEQ ID NO: 19 to SEQ ID NO: 28.

According to the method of the present invention the plasmid containing the genes involved in ß-carotene synthesis further contains the genes encoding a ß-carotene-ketolase and/or a ß-carotene-hydrolase. The gene expression of ketolase and hydrolase is performed under the control of inducible promoter, preferably *ara* promoter.

According to the method of the present invention the prokaryote cells are also transformed with a plasmid containing a apo-carotenoprotein genes.

As used herein the term "apo-carotenprotein" or "apo-protein" refers to a protein able to attach a carotenoid. Thus, the apo-carotenoprotein is the protein without the carotenoid.

According to the present invention the apo-carotenoprotein gene may be obtained from any organism selected in the group of any bacteria, any algae, any plant and any animal.

Preferably, the apo-carotenoprotein gene may be obtained from cyanobacteria selected from the group of strains comprising *Synechocystis sp., Anabaena sp. or Arthrospira sp.*

According to the invention the apo-carotenoprotein is a soluble protein.

Preferably, the apo-carotenoprotein is a soluble protein selected in the group comprising an apo-Orange Carotenoid Protein (OCP), an apo-Red Carotenoid Protein (RCP), an apo-AstaP, an apo-crustacyanin and an apo-glutathione s-transferasa like protein (GSTP1).

More preferably, the apo-carotenoproteins of the inventions are selected in the group consisting of apo-OCP and apo-RCP.

According to the more preferred embodiment of the present invention the gene encoding apo-OCP is obtained from bacteria selected in the group consisting in *Synechocystis, Arthrospira or Anabaena.*

Preferably, the genes sequences of the apo-OCP correspond to the sequences selected from the group comprising SEQ ID NO: 1, SEQ ID NO: 2 and SEQ ID NO: 3.

Preferably, the genes sequences of the apo-RCP correspond to the sequence SEQ ID NO: 4.

According to the present application the sequences of primers used for cloning the genes encoding apo-carotenoprotéine may be synthesized by any conventional method using any conventional skills.

Preferably, the sequences of primers used for cloning the genes encoding apo-carotenoprotéine correspond to SEQ ID NO: 29 to SEQ ID NO: 36.

According to a preferred embodiment of the present application, the gene encoding apo-carotenoprotein may be modified. Usually, the gene encoding apo-carotenoprotein is modified by introducing or deleted 9 to 45 nucleotides, preferably 24 to 30 nucleotides just after the first ATG of 5' end or just before the stop codon of said gene.

Particularly, these modifications are selected in the group of nucleotide sequences corresponding to SEQ ID NO: 8 to SEQ ID NO: 16 as shown in the sequences listing annexed herewith.

In all apo-carotenoproteins gene sequences a HisTag on N terminal (HisTagNter corresponding to SEQ ID NO: 17) or on C terminal (HisTagCter corresponding to SEQ ID NO: 18) may be introduced.

Preferably these gene sequences of apo-carotenoprotein are modified as shown in table 1.

**Table 1: Modifications of gene sequences encoding carotenoprotein**

| **Name of modifying sequence** | **Constructions of modified genes encoding apo-carotenoproteins** |
|---|---|
| NpCDFduet | ATG + SEQ ID NO: 8 + SEQ ID NO: 1 or 2 or 3 + HisTagCter |
| NC15 | ATG + SEQ ID NO: 9 + SEQ ID NO: 1 + HisTagCter |
| NC11 | ATG + SEQ ID NO: 10 + SEQ ID NO: 1 + HisTagCter |
| NC9 | ATG + SEQ ID NO: 11 + SEQ ID NO: 1 or 4 + HisTagCter |
| NC7 | ATG + SEQ ID NO: 12 + SEQ ID NO: 1 + HisTagCter |
| NC6 | ATG + SEQ ID NO: 13 + SEQ ID NO: 4 + HisTagCter |
| NC4 | ATG = SEQ ID NO: 14 + SEQ ID NO: 1 + HisTagCter |
| MIX15 | ATG + SEQ ID NO: 15 + SEQ ID NO: 1 + HisTagCter |
| C9 | ATG + SEQ ID NO: 16 + SEQ ID NO: 1 + HisTagCter |
| HisTagNter3aa (amino acid) | ATG + SEQ ID NO 17 + SEQ ID NO: 1 or 2 or 3 |
| HisTagCter | ATG + SEQ ID NO: 1 or 2 or 3 or 4 + SEQ ID NO: 18 + codon STOP |

According to the present invention the primer's sequences used for the modification of genes encoding apo-carotenoprotein and the ones used for amplification of these genes correspond to SEQ ID NO: 37 to SEQ ID NO: 68.

According to the present invention, the plasmids containing genes involved in carotenoid synthesis and the plasmids containing genes involved in apo-carotenoprotein synthesis contain different replication origins and different selective pressure.

As used in the invention the term "replication origin" refers to a particular sequence in a genome at which replication is initiated bidirectionally or unidirectionally.

As used herein the term "selective pressure" refers to the presence of genes giving resistance to different antibiotics to maintain the plasmids in the prokaryote cells.

According to one embodiment of the present Invention genes encoding enzymes involved in carotenoid synthesis and genes encoding an apo-carotenoprotein must be amplified prior to prokaryote cell transfection. Preferably, said genes are amplified by polymerase chain reaction (PCR). The primer's sequences for the amplification correspond to SEQ ID NOs: 29, 30, 31, 32, 33, 34, 35,36, 39, 40, 57, 58, 61 and 62.

According to the method of the present invention the transformed prokaryote cell are cultured in conditions allowing sequential gene expression.

To obtain a functional carotenoid-protein complex in high quantities, it is very important to cultivate the transfected prokaryote cells such as to induce in the first time the genes expression of genes encoding carotenoids and then, induce the gene expression of genes encoding apo-carotenoproteins.

According to one embodiment of the method of the present invention step b) of culturing the prokaryote cells comprises the following steps:
b1) expressing the genes encoding ß-carotene without specific induction;
b2) inducing the gene expression of ß-carotene ketolase and/or ß-carotene hydrolase at temperature ranges of 33 to 40° C, preferably of 35 to 38 and more preferably about 37°C;
b3) inducing the gene expression of apo-carotenoprotein at temperature ranges of 20 to 30°C, preferably of 22 to 28°Cand more preferably of 24 to 26°C.

According to a preferred embodiment of the invention, to obtain Synechocystis echinenone attached OCP, the step b2) of inducing ß- carotene ketolase genes expression *(CrtO)* is performed at 37°C and step b3) of inducing the gene expression of apo-OCP is performed at 28 ° C.

According to the method of the present invention isolating the carotenoid-protein complex in step c) may be performed by using any conventional column well in the art.

In respect to used tag, the skilled artisan may determinate easily a suitable conventional column to isolate the carotenoid-protein complex.

Preferably, to isolate the carotenoid-protein complex in step c) of the method of the present invention a Nickel column is used when the protein has a Histag.

In one aspect, the present invention relate to a carotenoid-protein complex (or carotenoprotein) obtained by the method of the present invention.

Preferably, said carotenoid-protein complex is a soluble carotenoid-protein complex.

More preferably, said carotenoid-protein complex is a soluble carotenoid-protein complex selected from the group comprising an orange carotenoid protein (OCP), a red carotenoid protein (RCP), AstaP, crustacyanin, glutathione s-transferasa like protein (GSTP1).

According to a preferred embodiment of the invention said carotenoid-protein complex is a soluble protein selected from the group comprising an orange carotenoid protein (OCP), a red carotenoid protein (RCP).

According to more preferred embodiment, the carotenoid-protein complex obtained by the method of the present invention is the OCP.

The Orange Carotenoid Protein (OCP) is a soluble protein that attaches a molecule of the ketocarotenoid 3'-hydroxy-echinenone. It is genetically encoded in many species and strains of cyanobacteria (Kirilovsky and Kerfeld, 2012). In cyanobacteria, the OCP is involved in the induction of a photoprotective mechanism that by increasing the thermal dissipation of the excess absorbed energy at the level of the phycobilisome, the cyanobacterial antenna, decreases the energy arriving at the photosynthetic reaction centers (Wilson et al., 2006)). The OCP is a photoactive protein. Strong light triggers the activation of the OCP inducing conformational changes in the carotenoid and the protein (Wilson et al., 2008). These changes convert the inactive dark orange OCP in the active red OCP. Only the active red OCP is able to bind the core of phycobilisomes. The bound redOCP quenches the absorbed light energy and the phycobilisome fluorescence with a high efficiency (Kirilovsky and Kerfeld, 2012). The OCP dissipates excess energy into heat.

Recently, it was demonstrated that OCP also protects cyanobacteria cells from oxidative stress by directly quenching singlet oxygen (Sedoud et al., 2014).

In one aspect the invention relates to modified apo-carotenoprotein genes encoding modified carotenoid-protein complex.

Preferably, the invention relate to a modified gene encoding apo- OCP or apo-RCP characterized in that it is modified by introducing 9 to 45 nucleotides preferably 24 to 30 nucleotides just after the first ATG of 5' end and/or just before the stop codon of said gene.

According to a preferred embodiment of the invention, the modifications are selected in the group of sequences comprising: SEQ ID NO: 8 to SEQ ID NO: 16 and SEQ ID NO 17 and 18 corresponding to HisTAgNter and HisTAgCter respectively.

Preferably, when the modified gene encodes an apo-OCP (orange carotenoid protein), the modifications are selected in the group of sequences comprising SEQ ID NOs: 8, 9, 10, 11, 12, 14, 15, 16, 17 and 18 (as shown on table 2).

Preferably, when the modified gene encodes an apo-RCP red carotenoid protein), the modifications are selected in the group of sequences comprising SEQ ID NOs: 9, 13 and 18 (as shown on table 2).

In one aspect the present invention relate to a vector, a plasmid or a host cell comprising a modified apo-carotenoprotein according to the invention.

Preferably, said vector according to the invention is a cloning or an expression vector.

The vector can be viral vector such as bacteriophages or non-viral vector such as plasmid.

The host cell according to the invention comprises a nucleic acid molecule according to the invention or vector according to the invention. The host cells according to the invention may be useful for synthesis of polypeptides according to the invention.

In another aspect the invention relate to modified OCP or RCP encoded by the modified apo-protein genes according to the invention.

The method of the present invention has the following advantages:
- allows to obtain between 10 to 30 mg carotenoprotein per liter of culture in only 4 days while in the prior art only 1.33 mg per liter after 3 weeks was obtained;
- allow to produce numerous natural protein-carotenoid complexes as well as create new ones;
- for the first time a water soluble carotenoid protein is obtained in prokaryote cell such as *E coli* with attached carotenoid, even with soluble carotenoid proteins wherein the isolated carotenoids are lipid-soluble and present only in membranes.

The present invention will be illustrated by following figures and examples.
Figure 1: SDS gels electrophoresis showing the proteins present in *E coli* cells overexpressing *Arthrospira* OCP and the proteins present in the different fractions during the *Arthrospira* OCP isolation from *E.coli* cells.
Figure 2: SDS gels electrophoresis showing the isolated *Synechocystis* OCPs containing different modifications in N-terminal and/or C-terminal of the protein.
Figure 3: shows the absorbance spectra of *Synechocystis, Arthrospira* and *Anabaena* ECN- OCPs isolated from *E.coli* cells (figure 3a) containing the additional sequence NpCDFduet in the N-terminal. Comparison of the ECN-OCPs isolated from *Synechocystis* and *E coli* cells (figure 3b).
Figure 4: shows the absorbance spectra of *Anabaena* (a) and *Synechocystis (b)* OCPs carrying canthaxanthin and those carrying the echinenone.
Figure 5: shows the absorbance spectra of the red *Synechocystis* OCPs isolated from *E coli* cells containing echinenone or canthaxanthin.
Figure 6: shows the kinetics of the photoconversion of different modified orange OCP from *Arthospira, Anabaena* and *Synechocystis* (figure 6a) and different modified orange OCP only from *Synechocystis* (figure 6b)to red OCP.
Figure 7: shows the induction of fluorescence quenching by different OCPs genes from *Synechocystis* having N-terminal modification (figure 7a) and by different OCP genes from *Arthospira, Anabaena* and *Synechocystis* having N-terminal modification (figure 7b).
Figure 8: shows EPR (electron paramagnetic resonance) signal of TEMPO (nitroxide radical of TEMPD-HCl 2,2,6,6-tetramethylpiperidone)in the absence and presence of OCP.
Figure 9: shows absorbance spectra of RCPs with different modifications in the N-terminal and binding echinenone (figure 9a) or cantaxanthin (figure 9b) or cantaxanthin and echinenone figure 9c).

### EXAMPLES

### I. Materials and methods

### 1) Amplification of crt genes encoding enzymes involved in carotenoid synthesis and of carotenoid binding proteins (ocp and rcp)

### • Carotenoid binding proteins (apo-carotenoproteins)

The *ocp* gene encoding the OCP from three different cyanobacteria strains *(slr1963* in *Synechocystis* PCC 6803 (SEQ ID NO:1); NIES39_N00720 in *Arthrospira platensis* (SEQ ID NO:2) and *all3149* in *Anabaena* PCC 7120 (SEQ ID NO:3)) and *rcp* gene *(all1123)* from *Anabaena* PCC 7120 (SEQ ID NO:4) were amplified by PCR.

Cyanobacteria genomes and plasmids containing the different *ocp* genes or the *rcp* genes with addition of a sequence (CACCACCACCACCACCAC, called HisTagCter) encoding six histidines in the 3'end followed by stop codon were used as templates.

For example such sequences are SEQ ID NO: 1- HisTagCter; SEQ ID NO:2 - HisTagCter; SEQ ID NO:3 - HisTagCter and SEQ ID NO: 4 - HisTagCter.

### • Crt Enzymes

The crtO gene (slr0088) from Synechocystis PCC 6803 SEQ ID NO:5) and the crtW gene (alr3189) from Anabaena PCC 7120 (SEQ ID NO: 6), both encoding for ß-carotene ketolases and the crtR gene (sll1468) of Synechocystis PCC 6803 (SEQ ID NO: 7) encoding for the ß -carotene hydrolase were amplified by PCR using cyanobacteria genomes as template and synthetic oligonucleotides as primers(SEQ ID NOs: 19/20 and 21/22 for crtO gene, SEQ ID NOs: 25/26 for crtR gene and SEQ ID NO: 27/28 for crtW gene) .

The iProof High Fidelity DNA Polymerase (from Bio Rad) was used to PCR amplification of the desired genes. The primers used herein.

### 2) Construction of plasmids: Cloning of different ocp, rcp and crt genes

### • Plasmid containing the genes encoding the enzymes needed to ß-carotene synthesis

Two different plasmids were used:
1) The construction of the Plasmid pAC-BETA, which contains the *crtB, crtE, crtI* and *crtY* genes from *Erwina herbicola* under the control of the promoter of *crtE* (gift of Prof Francis X.Cunningham) is described in (Cunningham et al., 1996).
2) The plasmid pACCAR16ΔcrtX containing the *crtB, crtE, crtI* and *crtY* genes from *Erwina uredovora* under the control of the promoter of *crtE,* (gift of Prof Sandmann) is described in (Misawa et al., 1995)These plasmids allow the synthesis of ß-carotene in the cell.

### • Plasmids containing the ß-carotene ketolases and ß-carotene hydrolases

The *crtO, crtW* and *crtR* genes were cloned in a modified plasmid pBAD/gIII A (from Invitrogen) which contains an arabinose inducible promoter (araBAD) and Ampicillin resistance. The expression of the *Crt* genes is thus enhanced by arabinose induction in the medium of culture.

CrtO and CrtW catalyse the conversion of ß-carotene to echinenone and canthaxanthin respectively and CrtR catalyzes the conversion of ß-carotene to zeaxanthin. The plasmid pBAD/gIII A was first modified to avoid the export of the recombinant protein into the periplasmic space of the cells. To this purpose the region encoding the "gene III signal sequence" was deleted. The primers used for the PCR mutagenesis were pBAD/gIIIAmut (F and R) corresponding to SEQ ID NO: 23 and SEQ ID NO: 24.

The plasmid pBAD/gIII A modified have been named pBAD. The Plasmid pBAD was digested with BgIII and EcoRI restriction enzymes to clone the crtO gene of Synechocystis or with Ncol and EcoRI restriction enzymes to clone the crtW gene of Anabaena PCC7120 and with Ncol and XhoI restriction enzyme to clone the crtR gene of Synechocystis. Primers CrtO (SEQ ID NOs: 19, 20, 21, 22), primers CrtR (SEQ ID NO: 25 and SEQ ID NO: 26) and primers CrtW (SEQ ID NO: 27 and SEQ ID NO: 28) were used to amplify crtO, crtR and crtW genes respectively. The resulting plasmids were named pBAD-CrtO, pBAD-CrtR and pBAD-CrtW. Theses primers and plasmids are shown on table 2 below.

**Table 2 : Plasmids and primers used for mutagenesis and cloning of genes encoding β-carotene ketolases and hydrolases**

| **Plasmids/Restriction enzyme** | **Primer's name** | **Primers SEQ ID NO:** |
|---|---|---|
| pBADgIII-CrtO BgIII/EcoRI | CrtO F | 19 |
| | CrtO R | 20 |
| pBAD-CrtO | | 21 |
| | | 22 |
| pBAD | pBAD/gIIIAmut F | 23 |
| | pBAD/gIIIAmut F | 24 |
| pBAD-CrtR NcoI/XhoI | CrtR F | 25 |
| | CrtR R | 26 |
| pBAD-CrtW NcoI/EcoRI | CrtW F | 27 |
| | CrtW R | 28 |

### • Plasmids containing the genes of carotenoid binding proteins

The *ocp* and *rcp* genes were cloned in a Plasmid pCDFDuet-1 (from Novagen). The plasmid pCDFDuet-1 contains T7lac promoters and Streptomycin/Spectinomycin resistance. The expression of the *ocp* and *rcp* genes is thus enhanced by IPTG induction in the medium of culture.

The pCDFduet-1 plasmid was digested with EcoRI and NotI to clone the different ocp genes (from Synechosystis PCC6803, Arthrospira Platensis PCC7345 and Anabaena PCC 7120). The primers OCPsynHistagNter15 (F and R) corresponding to SEQ ID NO: 29 and SEQ ID NO: 30 respectively were used to amplify the Synechocystis ocp gene (1104 nucleotides) using genomic DNA of Synechocystis PCC6803 as template. The primers OCPanaHisTagNter15 (F and R) corresponding to SEQ ID NO: 33 and SEQ ID NO: 34 respectively were used to amplify the Anabaena ocp gene (1076 nucleotides) using genomic DNA of Anabaena PCC 7120 as template. The primers OCParthroHistagNter15 (F and R) corresponding to SEQ ID NO: 31 and SEQ ID NO: 32 respectively were used to amplify the Arthrospira ocp gene (1355 nucleotides) using the plasmid pOF7345 as template (plasmid constructed by (Jallet et al., 2014)). The resulting PCR products were introduced into pCDFDuet-1 to create the pCDF-OCPsynHistagNter15, pCDF-OCPanaHistagNter15, pCDF-OCParthroHistagNter15 and plasmids. In the OCP isolated from E.coli cells carrying these plasmids, an extension of 15 amino acids will be present in the N-terminal of the OCP protein. This extension contains a His-tag of 6 His. (NpCDFduet extension corresponding to SEQ ID NO: 8).

**Table 3. Plasmids and primers used for cloning of genes encoding ocp genes**

| **Plasmids/Restriction enzymes** | **Primer's name** | **Primers SEQ ID NO:** |
|---|---|---|
| pCDF-OCPsynHisTagNter15 EcoRI/NotI | OCPsynHistagNter15 F | 29 |
| | OCPsynHistagNter15R | 30 |
| pCDF-OCParthroHisTagNter15 EcoRI/NotI | OCParthroHistagNter15 F | 31 |
| | OCParthroHistagNter15 R | 32 |
| pCDF-OCPanaHisTagNter15 EcoRI/NotI | OCPanaHistagNter15 F | 33 |
| | OCPanaHistagNter15 R | 34 |
| pCDFDuet-NC2-1123HIS NcoI/NotI | RCPanaCter F | 35 |
| | RCPanaCter R | 36 |

To obtain a C-terminal His-tagged *Synechocystis* OCP, first, the GCC sequence coding for Ala73 was mutated to GCG that also code for in alanine to abolish the Ncol site in the *Synechocystis ocp* gene sequence in the plasmid SK-OCPsyn-P2A-CterHisTagΔFRP (Wilson et al., 2008), to clone the OCPsyn- HisTagCter between NcoI and NotI sites.

Then, pCDFDuet-1 was digested with NcoI and NotI to excise the N-terminal extension containing the His-tag initially present in this plasmid. The ocp genes containing a C-terminal His-Tag from Synechosystis PCC6803, Arthrospira Platensis PCC7345 and Anabaena PCC 7120 were cloned in the plasmid. The primers OCPsynCter (F and R) corresponding to SEQ ID NO: 39 and SEQ ID NO: 40 respectively were used to amplify the ocp gene tagged in C-terminal domain from the plasmid pSK-OCPsyn-P2A-CterHisTagΔFRP- A73A (Wilson et al., 2008). The primers OCParthroCter (F and R) corresponding to SEQ ID NO: 57 and SEQ ID NO: 58 respectively were used to amplify the *ocp* gene from the plasmid pOF7345His (Jallet et al, 2014) which contains the *ocp* gene tagged in the C-terminal domain. The primers OCPanaCter (F and R) corresponding to SEQ ID NO: 61 and SEQ ID NO: 62 respectively were used to amplify the *ocp* gene from genomic DNA of *Anabaena* PCC 7120, the C-terminal His-tag was then added by PCR mutagenesis. The resulting PCR products were introduced into pCDFDuet-1 to create the pCDF-OCPsynCter, pCDF-OCParthroCter and pCDF-OCPanaCter plasmids. The OCP isolated from *E.coli* cells containing these plasmids, will contain a His-tag in their C-terminal (HisTagCter corresponding to SEQ ID NO: 18).

To obtain a C-terminal His-tagged RCP, the plasmid pCDFDuet-1 was digested with NcoI and NotI to excise the N-terminal extension containing the His-tag initially present in this plasmid. Then the the *rcp* gene from *Anabaena* PCC 7120 containing a C-terminal His-Tag was cloned in the plasmid. The primers RCPanaCter (F and R) corresponding to SEQ ID NO: 35 and SEQ ID NO: 36 respectively were used to amplify the *rcp* gene tagged in the C-terminal from the plasmid pPSBA2-1123HIS (constructed by Rocio Lopez Igual in the lab). The resulting PCR products were introduced into pCDFDuet-1 to create the pCDFDuet-NC2-1123HIS plasmid. The RCP isolated from *E.coli* cells containing this plasmid, will contain a His-tag in its C-terminal (SEQ ID NO: 18).

Table 4 shows the plasmids used in the present invention, their characteristics and their source.

**Table 4: All plasmids used in the present invention**

| **Plasmid** | **Characteristics** | **Source and reference** |
|---|---|---|
| pCDFDuet-1 | Commercially supplied overexpression plasmid vector using T7 promoter capable to carry two genes by two multi-cloning site, (SmR) | Novagen |
| pCDF-OCPsynHisTagNter15 | pCDFDuet-1 derivative, His-tag N-terminal OCP *Synechocystis PCC6803* gene | Performed in the present invention |
| pPSBA2-OCP/FRPsynC-terHisTAG | Plasmid constructed by Adjélé Wilson, which contains the OCP and FRP genes tagged in C-terminal from *Synechosystis* PCC6803 | Wilson et al, 2008 |
| pCDF-OCPsynCter | pCDFDuet-1 derivative, His-tag C-terminal OCP Synechocystis PCC6803 gene | Performed in the present invention |
| pOF7345 | Plasmid constructed by Denis Jallet, which contains the OCP and FRP genes of Arthrospira Platensis PCC7345 | Jallet et al, 2014 |
| pCDF-OCParthroHisTagNter15 | pCDFDuet-1 derivative, His-tag N-terminal OCP Arthrospira Platensis PCC7345 gene | Performed in the present invention |
| pOF7345His | Plasmid constructed by Denis Jallet, which contains the OCP and FRP genes tagged in C-terminal, of Arthrospira Platensis PCC7345 | Jallet et al, 2014 |
| pCDF-OCParthroCter | pCDFDuet-1 derivative, His-tag C-terminal OCP Arthrospira Platensis PCC7345 gene | Performed in the present invention |
| pCDF-OCPanaHisTagNter15 | pCDFDuet-1 derivative, His-tag N-terminal OCP Anabaena PCC 7120 gene | Performed in the present invention |
| pCDF-OCPanaCter | pCDFDuet-1 derivative, His-tag C-terminal OCP Anabaena PCC 7120 gene | Performed in the present invention |
| pCDFDuet-NC2-1123HIS | pCDFDuet-1 derivative, His-tag C-terminal RCP Anabaena PCC 7120 gene | Performed in the present invention |
| pBAD/gIII A | Commercially supplied overexpression plasmid vector using araBAD promoter, (AmpR) | Invitrogen |
| pBAD | pBAD/gIII A derivative, Δ geneIII signal sequence | Performed in the present invention |
| pBAD-CrtO | pBAD derivative, CrtO Synechocystis PCC6803 gene | Performed in the present invention |
| pBAD-CrtR | pBAD derivative, CrtR Synechocystis PCC6803 gene | Performed in the present invention |
| pBAD-CrtW | pBAD derivative, CrtW Anabaena PCC7120 gene | Performed in the present invention |
| pAC-BETA | *P15A ori,* Cm(CmR), pACYC184 derivative, *E.herbicola* crt genes | Cunningham et al. (1996) |
| pACCAR16□crtX | *P15A ori,* Cm(CmR), pACYC184 derivative, *E.uredovora* crt genes | Misawa et al, 1995 |

### Modifications of the sequences of ocp and rcp genes

To increase the yield of carotenoid-OCP complexes in *E.coli* cells, modifications were introduced in the sequence of the *ocp* gene. Several modifications were tested in the *Synechocystis ocp* gene and then the best ones were introduced in the *Arthrospira* and *Anabaena ocp* genes. Modifications were also introduced in the *Anabaena rcp* gene. The sequences added after the first *ATG* of the gene correspond to SEQ ID NOs: 9, 10, 11, 12, 14, 15 and 16 (respectively called NC15, NC11, NC9, NC7, NC4, Mix15 and C9). They were introduced by directed mutagenesis, using the pCDF-OCPSynCter plasmid as template and primers corresponding to SEQ ID NOs: (41 and 42), (45 and 46), (47 and 48), (49 and 50), (51 and 52), (43 and 44), (53 and 54) respectively The modification HisTagNter3aa was introduced by directed mutagenesis using the pCDF-OCPSynHisTagNter15 plasmid as template and primers corresponding to SEQ ID NOs: 55 and 56 causing the deletion of a part of the N-terminal prolongation. The modification HisTagNter3aa was also introduced to the ocp genes of *Arthrospira* and *Anabaena* using the pCDF-OCParthroHisTagNter15 and pCDF-OCPanaHisTagNter15 plasmids as templates and the primers corresponding to SEQ ID NOs: 59 and 60 and SEQ ID NOs: 63 and 64 respectively.

The modifications corresponding to SEQ ID NOs: 11 and 13 (called NC9 and NC6 respectively) were introduced to the *rcp* gene using the pCDFDuet-NC2-1123HIS plasmid as template and the primers corresponding to SEQ ID NOs: 65 and 66 and SEQ ID NOs: 67 and 68 respectively.

Table 5 shows the modified plasmids comprising a modified *ocp* genes.

**Table 5: Modified plasmids comprising modified ocp genes performed and used in this invention**

| **Plasmid modified** | **Characteristics** | **Source and reference** |
|---|---|---|
| pCDF-OCPsynCterNterNC15 | Mutagenesis using pCDF-OCPsynCter | Performed in the invention |
| pCDF-OCPsynCterNterNC11 | Mutagenesis using pCDF-OCPsynCter | Performed in the invention |
| pCDF-OCPsynCterNterNC9 | Mutagenesis using pCDF-OCPsynCter | Performed in the invention |
| pCDF-OCPsynCterNterNC7 | Mutagenesis using pCDF-OCPsynCter | Performed in the invention |
| pCDF-OCPsynCterNterNC4 | Mutagenesis using pCDF-OCPsynCter | Performed in the invention |
| pCDF-OCPsynCterNterMIX15 | Mutagenesis using pCDF-OCPsynCter | Performed in the invention |
| pCDF-OCPsynCterNterC9 | Mutagenesis using pCDF-OCPsynCter | Performed in the invention |
| pCDF-OCPsynNter3aa | Mutagenesis using pCDF-OCPsynHisTagNter15 | Performed in the invention |
| pCDF-OCParthroNter3aa | Mutagenesis using pCDF-OCParthroHisTagNter15 | Performed in the invention |
| pCDF-OCPanaNter3aa | Mutagenesis using pCDF-OCPanaHisTagNter15 | Performed in the invention |
| pCDF-NC9-1123HIS | Mutagenesis using pCDFDuet-NC2-1123HIS | Performed in the invention |
| pCDF-NC6-1123HIS | Mutagenesis using pCDFDuet-NC2-1123HIS | Performed in the invention |

### 3) The E.coli strains producing OCP and RCP

Two different bacterial strains were used for gene cloning and gene expression:
1) *E.coli* XL10-Gold from Agilent (TetrD(*mcrA*)*183 D(mcrCB-hsdSMR-mrr)173 endA1 supE44 thi-1 recA1 gyrA96 relA1 lac* Hte [F' *proAB lacl*qZD*M15* Tn*10* (Tetr) Amy Camr]) was used for gene cloning and grown in LB medium at 37°C, and
2) *E.coli* BL21-Gold (DE3) from Agilent (F- *ompT hsdS(rB -* mB-) *dcm+* Tetr *gal* λ(DE3) *endA* Hte) was used for OCP production.

*E.coli* strains producing OCP or RCP constructed in this invention are all derivatives of E coli BL21-Gold (DE3).

BL21 cells were transformed with the pAC-BETA, pBAD-CrtO (or pBAD-CrtW or pBAD-CrtR) and pCDF-OCP or pCDF-RCP plasmids. The latter plasmids contain WT or modified sequences of *ocp* or *rcp* genes (table 5).

**Table 6: Strains of E. coli producing different carotenoids created in this invention and plasmids used to create these strains used in the invention.**

| **Strains** | **Plasmid contents** |
|---|---|
| BL21-pßcarotene | pAC-BETA (Francis X.Cunningham) |
| BL21-pßcarotene | pACCAR16ΔCrtX (Sandmann) |
| | pAC-BETA |
| BL21echi | pBAD-CrtO |
| | pAC-BETA |
| BL21echiBIS | pCDF-CrtO |
| | pAC-BETA |
| BL21zea | pBAD-CrtR |
| | pAC-BETA |
| BL21cantha | pBAD-CrtW |

**Tableau 7: E coli strains containing ocp and rcp genes (obtained from different microorganisms cloned in pCDF-Duet plasmids) and containing crtO or crtW or CrtR genes cloned in pBAD plasmids.**

| **E coli containing SYNECHOCYSTIS OCP** | **PLASMID CONTENT** |
|---|---|
| BL21echi-OCPsynHisTagNter15 | pAC-BETA |
| | pBAD-CrtO |
| | pCDF-OCPsynHisTagNter15 |
| BL21echi-OCPsynCter | pAC-BETA |
| | pBAD-CrtO |
| | pCDF-OCPsynCter |
| BL21echi-OCPsynNter3aa | pAC-BETA |
| | pBAD-CrtO |
| | pCDF-OCPsynNter3aa |
| BL21echi-OCPsynCterNter15 | pAC-BETA |
| | pBAD-CrtO |
| | pCDF-OCPsynCterNter15 |
| BL21echi-OCPsynCterNter11 | pAC-BETA |
| | pBAD-CrtO |
| | pCDF-OCPsynCterNter11 |
| BL21 echi-OCPsynCterNter9 | pAC-BETA |
| | pBAD-CrtO |
| | pCDF-OCPsynCterNter9 |
| BL21 echi-OCPsynCterNter7 | pAC-BETA |
| | pBAD-CrtO |
| | pCDF-OCPsynCterNter7 |
| BL21 echi-OCPsynCterNter4 | pAC-BETA |
| | pBAD-CrtO |
| | pCDF-OCPsynCterNter4 |
| | pAC-BETA |
| BL21echi-OCPsynCterC9 | pBAD-CrtO |
| | pCDF-OCPsynCterNterC9 |
| BL21echi-OCPsynCterMIX15 | pAC-BETA |
| | pBAD-CrtO |
| | pCDF-OCPsynCterNterMIX15 |
| BL21zea-OCPsynHisTagNter15 | pAC-BETA |
| | pBAD-CrtR |
| | pCDF-OCPsynHisTagNter15 |
| BL21zea-OCPsynCter | pAC-BETA |
| | pBAD-CrtR |
| | pCDF-OCPsynCter |
| BL21cantha-OCPsynCter | pAC-BETA |
| | pBAD-CrtW |
| | pCDF-OCPsynCter |
| BL21cantha-OCPsynNter3aa | pAC-BETA |
| | pBAD-CrtW |
| | pCDF-OCPsynNter3aa |

| E coli containing ANABAEANA OCP | PLASMID CONTENT |
|---|---|
| BL21 echi-OCPanaHisTagNter15 | pAC-BETA |
| | pBAD-CrtO |
| | pCDF-OCPanaHisTagNter15 |
| BL21 echi-OCPanaCter | pAC-BETA |
| | pBAD-CrtO |
| | pCDF-OCPanaCter |
| BL21echi-OCPanaNter3aa | pAC-BETA |
| | pBAD-CrtO |
| | pCDF-OCPanaNter3aa |
| BL21cantha-OCPanaCter | pAC-BETA |
| | pBAD-CrtW |
| | pCDF-OCPanaCter |
| BL21cantha-OCPanaNter3aa | pAC-BETA |
| | pBAD-CrtW |
| | pCDF-OCPanaNter3aa |

| E coli containing ARTHROSPIRA OCP | PLASMID CONTENT |
|---|---|
| BL21echi- OCParthroHisTagNter15 | pAC-BETA |
| | pBAD-CrtO |
| | pCDF-OCParthroHisTagNter15 5 |
| BL21echi-OCParthroCter | pAC-BETA |
| | pBAD-CrtO |
| | pCDF-OCParthroCter |
| BL21echi-OCParthroNter3aa | pAC-BETA |
| | pBAD-CrtO |
| | pCDF-OCParthroNter3aa |
| BL21cantha-OCParthroCter | pAC-BETA |
| | pBAD-CrtW |
| | pCDF-OCParthroCter |
| BL21cantha-OCParthroNter3aa | pAC-BETA |
| | pBAD-CrtW |
| | pCDF-OCParthroNter3aa |

| **E coli containing ANABAEANA RCP** | PLASMID CONTENT |
|---|---|
| BL21cantha-RCPanaCter | pAC-BETA |
| | pBAD-CrtW |
| | pCDFDuet-NC2-1123HIS |
| BL21cantha-RCPanaCterNterNC6 | pAC-BETA |
| | pBAD-CrtW |
| | pCDF-NC6-1123HIS |
| BL21cantha-RCPanaCterNterNC6 | pAC-BETA |
| | pBAD-CrtW |
| | pCDF-NC9-1123HIS |

### 4) Production of recombinant OCP

*Synechocystis* echinenone (ECN)-OCP was obtained by following steps:
1) 1 ml of stock glycerol (600µl E coli cells + 400µl glycerol) is diluted in 200 ml TB (containing 50 µg/ml chloramphenicol, 50 µg/ml ampicillin, 50 µg/ml streptomycin)
2) Incubation at 37 °C for 3-4 hours until arrive to OD600= 0.8.
3) When the culture is at OD600= 0.8, 0.02 % Arabinose is added to induce the transcription of the *CrtO* gene. The culture in the presence of arabinose is incubated overnight at 37 °C.
4) In the next morning; 800 ml of fresh TB are added to the 200 ml *E.coli* culture.
5) The cells are incubated at 37°C in the presence of 0.02% Arabinose until OD600= 1-1.5.
6) Addition of IPTG to a final concentration of 0.2mM IPTG to the culture at OD higher than 1.
7) Incubation overnight in the presence of 0.02% arabinose and 0.2mM IPTG at 28°C. (For *rcp* this incubation must be done at 18-20 °C)
8) In the next morning the frozen pellet of cells is resuspended in the lyse buffer containing 40mM Tris pH 8, 10% glycerol and 300mM NaCl. After resupension, 1 mM [EDTA, PMSF/DMSO,caproïc acid, benzamidine acid] and Dnase were added in the lyses buffer and the cells were broken in dim light using a "French Press" (twice at 750 psi). The broken cells were centrifuged at 20000 rpm for 30 min at 4°C and the obtained supernatant was then kept for OCP purification on nickel column. The supernatant was loaded on a column of nickel Probond resin (Invitrogen). After column washing (15 and 60 mM Imidazol) the purified OCP was eluted with 200 mM Imidazol.

### 5) Methods of characterisation of OCP

### Absorbance measurements

Absorbance spectra and the kinetics of orange to red OCP photoconversion and dark red to orange OCP reconversion were measured in a Specord S600 (Analyticjena) spectrophotometer. The kinetics were monitored during illumination of the OCP with 5000 µmol photons m⁻² s⁻¹ of white light and after turn-off the light at 18°C.

### Quantification of protein and carotenoid

The concentration of proteins was measured by the Bradford method and the concentration of carotenoid was deduced from the absorbance spectrum using the absorption coefficient of echinenone A1^{%}= 2158. The concentrations of protein and carotenoid are calculated in µM and the ratio will give the % of apo-OCP attached to the carotenoid molecule.

### Gel electrophoresis

The different steps of the OCP and RCP purification were followed by gel electrophoresis using SDS-PAGE on 12% polyacrylamide in a Tris/MES system (Kashino et al., 2001).

### Measurement of carotenoid content in E.coli cells and OCP

To determine the carotenoid content of the cells, an aliquot of *E.coli* cells was harvested by centrifugation at 14000 rpm for 1 min and washed once with water. The cell pellets were resuspended in 500µl of acetone and incubated at room temperature for 15 min in the dark. The tubes were centrifuged at 14000 rpm for 15 min, and the supernatant containing carotenoids was transferred to a new tube.

To determine the carotenoid content of OCP, OCP was concentrated with centrifugal filter units (Millipore). The carotenoid was extracted by acetone. After drying carotenoid extracted was resupended in 100% di-ether/100µl ethanol Carotenoid content was analysed by thin layer chromatography (TLC) with 90% petroleum ether/10% ethanol as mobile phase.

The carotenoid content of OCPs and RCPs was also analysed by High-Performance Liquid Chromatography (HPLC) and Mass spectrometry as described in (Punginelli et al., 2009).

### Fluorescence measurements

Fluorescence quenching and recovery were monitored with a pulse amplitude modulated fluorometer (101/102/103-PAM; Walz, Effelrich, Germany). All measurements were carried out in a stirred cuvette of 1 cm diameter. Typically, the fluorescence quenching was induced by 870 µmol photons m⁻² s⁻¹ of blue-green light (400-550 nm). All the reconstitution experiments were carried out at 23°C. The phycobilisomes are first illuminated and then redOCP (previously illuminated by strong white light) is added and the decreased of fluorescence is monitored.

### ¹O₂ detection by EPR spin trapping.

The formation of a nitroxide radical, which is a paramagnetic species arising from the interaction of TEMPD with ¹O₂, was measured by EPR in the absence or presence of different amount of purified proteins in buffer Tris-HCl 100 mM pH 8.0, TEMPD-HCl 100 mM and methylene blue (10 µM). The samples were illuminated for 3 min with strong white light (1000 µmol quanta m⁻² s⁻¹).

### II. Results

### 1) Characteristics of isolated OCPs

### OCP isolation

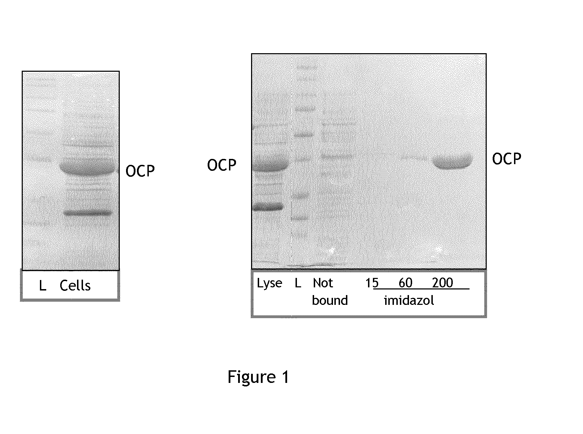

The OCPs were isolated using affinity Nickel-chromatography. The isolation was followed by gel electrophoresis. Figure 1 shows one example with the purification of ECN-Arthrospira OCP. The soluble proteins present in *E coli* cells and the proteins present in the different washings (15, 60 and 200 µM imidazole) of the Nickel column are shown. The last lane in each figure shows the purified protein, eluted with 200 µM imidazole (figure 1).

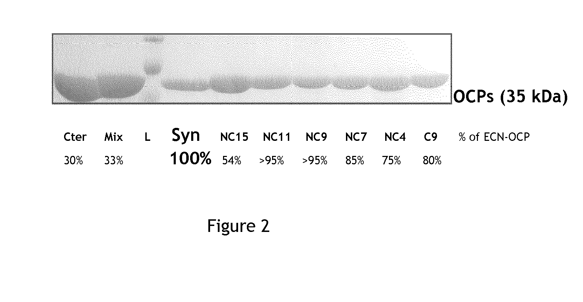

The isolated *Synechocystis* OCPs containing different modifications in the N-terminal and/or C-terminal of the protein are shown on Figure 2 wherein in the SDS-gel of total proteins of E coli cells a big band at 35kDa is observed corresponding to the OCP. The OCPs containing small quantities of echinenone are seen as bigger bands. In each lane was loaded the same quantity of carotenoid

### Yield of OCP-carotenoid complexes

The yield of OCP- carotenoid complex obtained by the method of the present invention is shown on table 8.

**Table 8: Examples of yield of OCP obtained from E coli cells when the overexpression of the ocp gene was done at 28°C**

| OCP produced in E.coli | OCP-carotenoid (%) | Average quantity of total OCP(holo + apo-OCP) (mg/L) |
|---|---|---|
| OCP Synechocystis PCC 6803 | | |
| OCPsynNter15-echi | 30-50 | 18-22 |
| OCPsynCter-echi | 20-40 | 4-6 |
| OCPsynNter+3aa-echi | >95 | 30-35 |
| OCPsynNter+3aa-cantha | 75-85 | 8-12 |
| OCPsynCterNterMIX15-echi | 30-40 | 20-22 |
| OCPsynCterNterNC15-echi | 45-55 | 18-21 |
| OCPsynCterNterNC11-echi | >95 | 9-11 |
| OCPsynCterNterNC9-echi | >95 | 15-17 |
| OCPsynCterNterNC7-echi | 75-85 | 19-21 |
| OCPsynCterNterNC4-echi | 70-80 | 8-10 |
| OCPsynCterNterC9-echi | 75-85 | 20-22 |
| OCP Arthrospira PCC 7345 | | |
| OCParthroNter15-echi | 30-40 | 18-22 |
| OCParthroCter-echi | 10-25 | 14-16 |
| **OCParthroCter-cantha** | **50-60** | **30-35** |
| OCParthroNter+3aa-echi | 30-40 | 24-26 |
| OCParthroNter+3aa-cantha | 60-65 | 28-30 |
| **OCP *Anabaena* PCC 7120** | | |
| OCPanaNter15-echi | 10-30 | 4-6 |
| OCPanaCter-echi | - | - |
| **OCPanaCter-cantha** | **50-60** | **60-70** |
| OCPanaNter+3aa-echi | 50-60 | 50-60 |
| OCPanaNter+3aa-cantha | 40-50 | 60-70 |

Production of OCP from modified *E.coli* strains with apo-OCP gene originated from several cyanobacteria has been compared. The results are shown on table 8. All the Synechocystis OCP contained an C-terminal His-tag with the exemption of the OCPsynNter+3aa-echi OCP and OCPsynNter15-echi OCP. The *Synechocystis* OCPs non-containing N-terminal prolongations attached very badly the carotenoid protein: only 20-35% of the isolated OCP contained a carotenoid molecule.

In addition, the yield of apo-OCP production was lower in the absence of the N-terminal prolongation. When the N-terminal prolongation contains 15 aa, the stability of the carotenoid binding depends on the sequence of these amino acids : The % of ECN-OCP varied from 30 to 55%. N-terminal extensions of 8 to 10 aa just after the first methionine gave the better % of ECN-OCP and the better yield. The amino acid composition of the extension had little effect.

In conclusion, modification of the apo-OCP is necessary to obtain high quantities of carotenoid-protein complexes with antioxidant activity. The protein without carotenoid has not this activity.

The same effect was observed with *Arthrospira* OCP and with the *Anabaena* OCP. For which only 10% of the protein binds the echinenone in the absence of the N-terminal prolongation. Large quantities of *Arthrospira* and *Anabaena* OCPs binding a carotenoid were obtained in the presence of canthaxanthin. For this the *E coli* cells were transformed with a pBAD-*CrtW* plasmid.

### Absorbance Spectra and Photoconversion

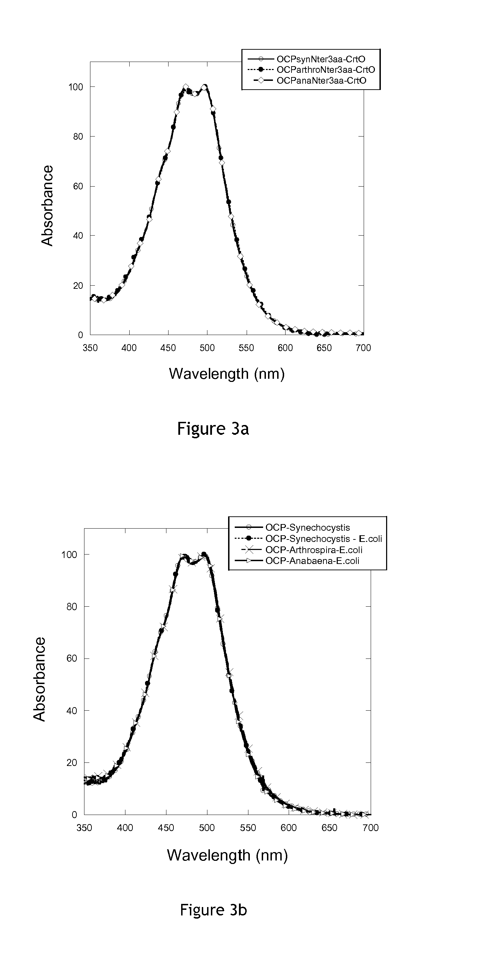

Figure 3 shows the spectra of three OCPs isolated from *E coli* cells containing echinenone. The figure also shows the spectrum of the echinenone- OCP isolated from *Synechocystis* cells. All the spectra were identical. Moreover all the isolated OCPs isolated from E coli cells presented the same spectra independently of the modification they carried.

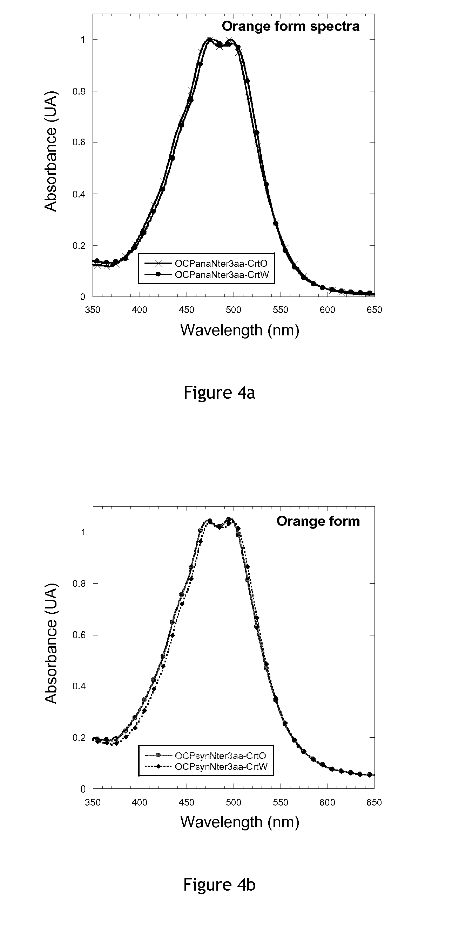

In addition, all the OCPs isolated from *E coli* cells carrying echinenone or canthaxanthine are photoactive like the OCP isolated from *Synechocystis.* In darkness, they are orange and upper illumination they become red (Figure 4).

Figure 4 shows that the spectre of the OCPs carrying canthaxanthin is slightly different than those carrying the echinenone. They are slightly red shifted.

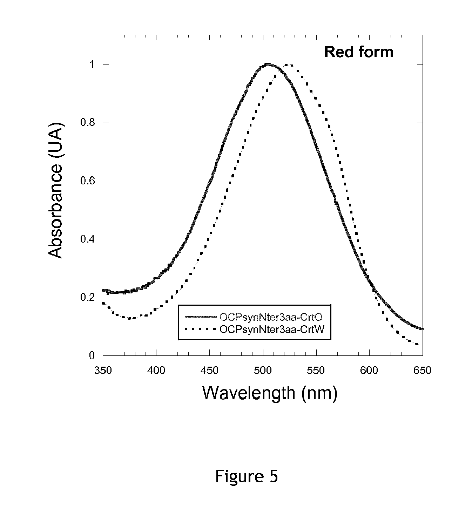

The spectra of the red OCPs containing canthaxanthin were red shifted compared to those containing echinenone (Figure 5).

The photoconversion of orange OCP to red OCP was evaluated by measuring the changes of absorbance at 550 nm (1= 100% red form; 0= 100% orange form). The increase in absorbance follows the accumulation of the red form.

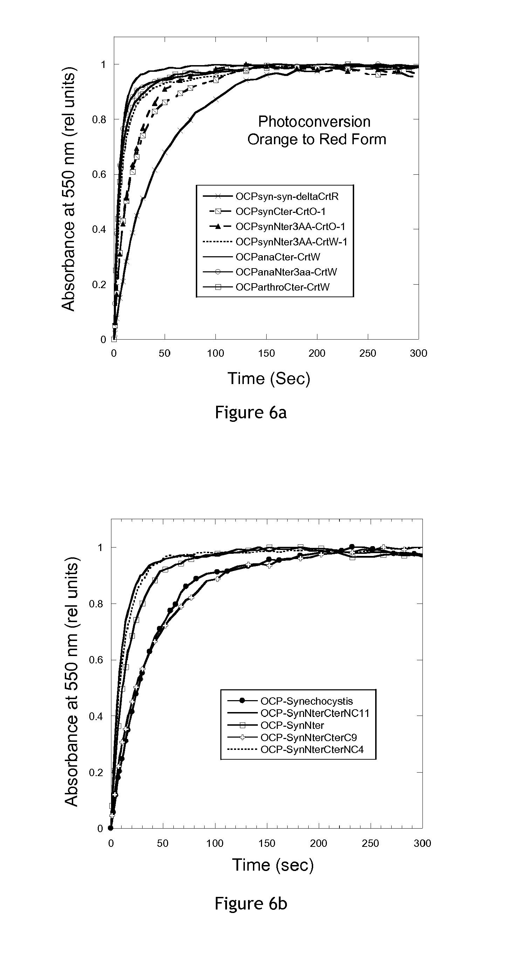

The kinetics of photoconversion are shown in Figure 6. Almost all of the OCPs isolated from *E coli* cells were converted to the red form faster than the OCP isolated from *Synechocystis.* The OCPs containing canthaxanthin were converted even faster (Figure 6).

### Induction of phycobilisome fluorescence quenching

In cyanobacteria cells, the main activity of the OCP is the induction of excess energy dissipation as heat. The activated red OCP binds the phycobilisomes and thermally dissipates the energy absorbed by them. This is accompanied by a concomitant decrease of fluorescence. The capacity of OCPs to bind to phycobilisomes and to dissipate excess energy can be measured *in vitro* using a reconstitution system developed by inventor's laboratory (Gwizdala et al., 2011) using isolated *Synechocystis* phycobilisomes and isolated OCPs.

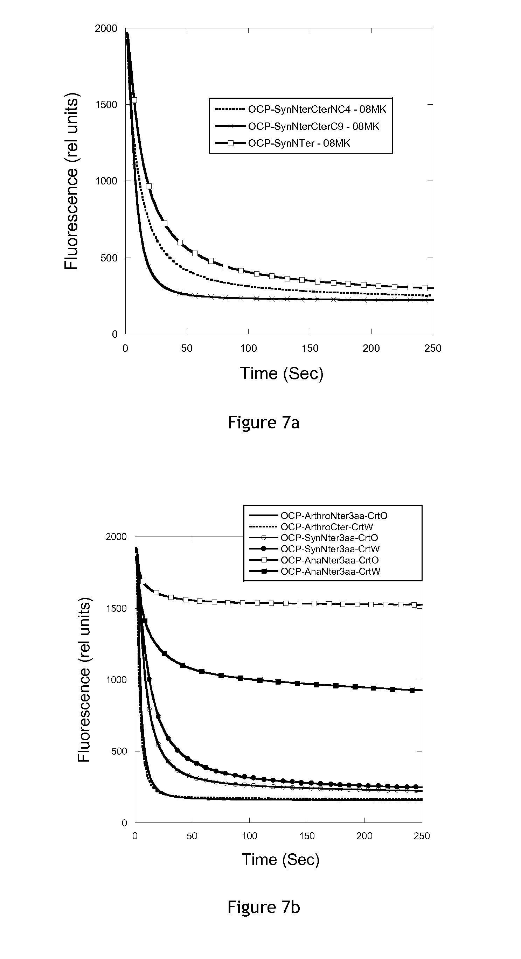

The phycobilisomes were illuminated 30sec with high intensities of blue-green light and then photoactivated red OCPs were added and the decrease of fluorescence was followed in a PAM fluorometer during 300 sec. Examples of induction of fluorescence quenching by different red OCPs are shown on figure 7.

Figure 7 shows the decrease of fluorescence induced by binding of red OCPs to the phycobilisomes. It was observed that the different modifications on the N-terminal side of the OCP provoke slight differences in the kinetics of quenching. The modifications can accelerate or decelerate the rates of quenching. Nevertheless all the *Synechocystis* OCPs with the tested modifications were able to induce a large phycobilisome quenching.

*Arthrospira* OCPs carrying echinenone or canthaxanthin are able to induce a very fast fluorescence quenching (figure 7b). Anabaena OCPs also induced fluorescence quenching. The lower capacity to induce fluorescence quenching of Anabaena OCPs is also observed in an Anabaena OCP carrying hydroxyechinenone and obtained from overexpression in Synechocystis.

### Quenching of Singlet Oxygen

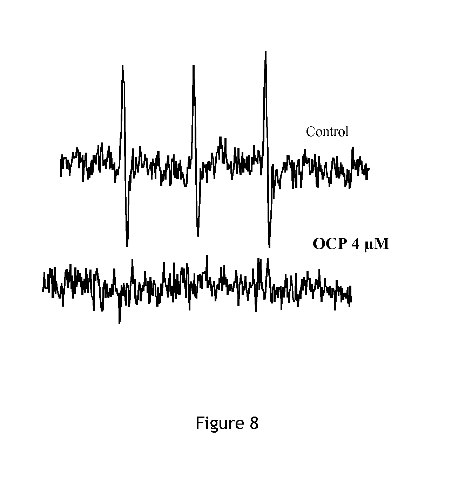

The singlet oxygen (¹O₂) quenching activity of OCPs was measured *in vitro* as described in Sedoud et al, 2014. Electron paramagnetic resonance (EPR) spin trapping was applied for ¹O₂ detection using TEMPD-HCl (2,2,6,6-tetramethyl-4-piperidone). When this nitrone reacts with ¹O₂, it is converted into the stable nitroxide radical, which is paramagnetic and detectable by EPR spectroscopy. The production of ¹O₂ was induced by illumination of the photosensitizer methylene blue. Figure 8 shows the typical EPR signal of the nitroxide radical obtained after 3 min illumination (1000 µmol quanta m⁻² s⁻¹) of a solution containing methylene blue and TEMPD-HCl. When this incubation was realized in the presence of 4 µM OCP, almost no EPR signal was detected indicating that OCP acts as a ¹O₂ quencher. The concentration of OCP that decreased 50% the EPR signal (I₅₀) was about 1-1.5 µM for all OCPs tested.

### 1) Characteristics of isolated RCPs

### Yield of RCP-carotenoid complexes

In *Anabaena* PCC 7120 there exist 4 genes coding only *rcp* genes with a high similarity to the N-terminal domain of the OCP (Sedoud et al., 2014). One of them, *all1123*, with different modifications in the 5' end of the gene, was overexpressed in *E coli* cells in the presence of echinenone or canthaxanthin.

The *rcp* gene was overexpressed in E coli cells at 28°C with a sequence coding 6 histidines in the 3'end with or without modifications in the 5' end. When the gene was induced in E coli cells producing echinenone, less than 5% of the isolated RCP was attached to a carotenoid molecule. Addition of amino acids in its N-terminal did not increase the attachment of the carotenoid. Induction of the rcp gene at lower temperatures did not improve the yield. When the rcp gene was induced in the presence of cantaxanthin, the yield of holo-RCP largely increased arriving to 15-20%. In this case, the induction of the *rcp* gene at lower temperatures (for example, 20°C) increased even more the yield of holo-RCP (30-40%).

**Table 9: shows: % of carotenoid-RCPs obtained from E coli cells at 28 and 20 °C.**

| **RCP (All1123) produites chez *E.coli*** | | **RCP-caroténoide (%)** |
|---|---|---|
| NC2-all1123-ECN | 28°C | 1-3% |
| NC9-all1123-ECN | 28°C | 1-3 % |
| C6-all1123-ECN | 28°C | 1-3 % |
| C6-all1123-canta | 28°C | 15-20 % |
| C6-all1123-canta | 20°C | 30-40 % |
| NC2-all1123-canta | 20°C | 30-40 % |

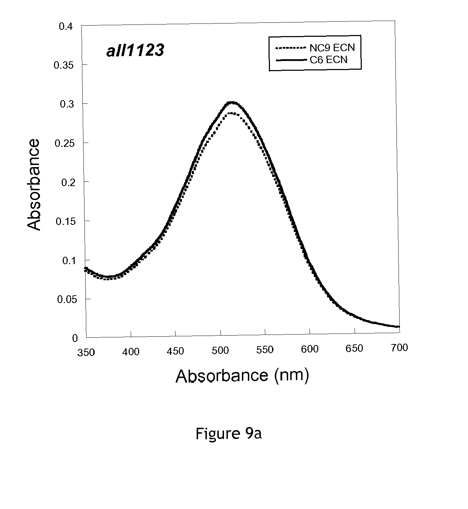

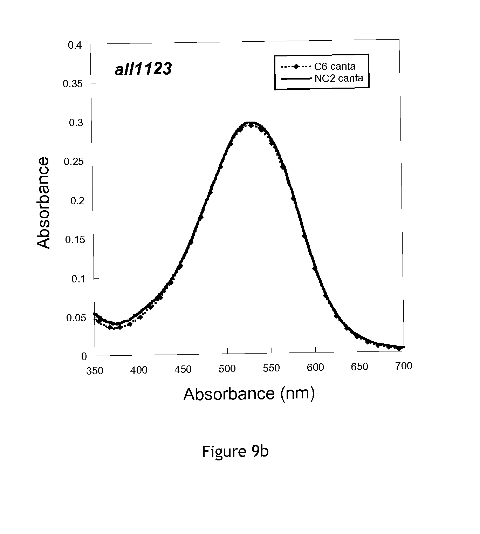

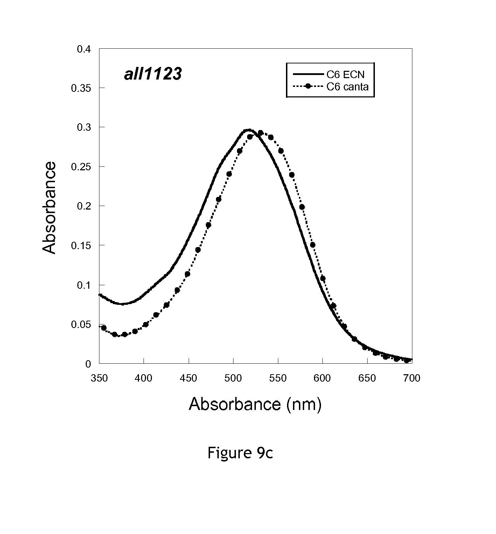

The absorbance spectra of the RCP proteins isolated from *Synechocystis* presented peaks at 498 and 530 nm and a shoulder at 470 nm because they bind mixoxanthophylls while that isolated from *Anabaena* binding canthaxanthin presented a large pic with a maximum at 530 nm similar to that shown in Figure 9.

The His-tagged protein of 20 kDa was isolated from E coli cells using a Nickel column as previously described for the isolation of the OCP. The isolated proteins were red. The spectra of the RCPs are described in Figure 9. The spectra of the cantaxanthin RCPs were red shifted compared to those of the echinenone RCPs.
Armstrong G (1997) Genetics of Eubacterial Carotenoid Biosynthesis: A Colorful Tale Annu Rev Microbiol 1997, 51 : 629-659
Bhosale P, Bernstein PS (2005) Synergistic effects of zeaxanthin and its binding protein in the prevention of lipid membrane oxidation. Biochim Biophys Acta 1740: 116-121
Cunningham FX, Jr., Pogson B, Sun Z, McDonald KA, DellaPenna D, Gantt E (1996) Functional analysis of the beta and epsilon lycopene cyclase enzymes of Arabidopsis reveals a mechanism for control of cyclic carotenoid formation. Plant Cell 8: 1613-1626
Giuffra E, Cugini D, Croce R, Bassi R (1996) Reconstitution and pigment-binding properties of recombinant CP29. Eur J Biochem 238: 112-120
Gwizdala M, Wilson A, Kirilovsky D (2011) In vitro reconstitution of the cyanobacterial photoprotective mechanism mediated by the Orange Carotenoid Protein in Synechocystis PCC 6803. Plant Cell 23: 2631-2643
Jallet D, Thurotte A, Leverenz RL, Perreau F, Kerfeld CA, Kirilovsky D (2014) Specificity of the cyanobacterial orange carotenoid protein: influences of orange carotenoid protein and phycobilisome structures. Plant Physiol 164: 790-804
Kashino Y, Koike H, Satoh K (2001) An improved sodium dodecyl sulfate-polyacrylamide gel electrophoresis system for the analysis of membrane protein complexes. Electrophoresis 22: 1004-1007
Kawasaki S, Mizuguchi K, Sato M, Kono T, Shimizu H (2013) A novel astaxanthin-binding photooxidative stress-inducible aqueous carotenoprotein from a eukaryotic microalga isolated from asphalt in midsummer. Plant Cell Physiol 54: 1027-1040
Kerfeld CA, Sawaya MR, Brahmandam V, Cascio D, Ho KK, Trevithick-Sutton CC, Krogmann DW, Yeates TO (2003) The crystal structure of a cyanobacterial water-soluble carotenoid binding protein. Structure 11: 55-65
Kirilovsky D, Kerfeld CA (2012) The orange carotenoid protein in photoprotection of photosystem II in cyanobacteria. Biochim Biophys Acta 1817: 158-166
Misawa, Nakagawa M, Kobayashi K, Yamano S, Izawa Y, Nakamura K and Harashima K, (1990) Elucidation of the Erwinia uredovora carotenoid biosynthetic pathway by functional analysis of gene products expressed in E coli. J Bacteriol, 172, 6704-6712
Misawa N, Satomi Y, Kondo K, Yokoyama A, Kajiwara S, Saito T, Ohtani T, Miki W (1995) Structure and Functional-Analysis of a Marine Bacterial Carotenoid Biosynthesis Gene-Cluster and Astaxanthin Biosynthetic-Pathway Proposed at the Gene Level. Journal of Bacteriology 177: 6575-6584
Punginelli C, Wilson A, Routaboul JM, Kirilovsky D (2009) Influence of zeaxanthin and echinenone binding on the activity of the Orange Carotenoid Protein. Biochim Biophys Acta 1787: 280-288
Sedoud A, Lopez-Igual R, Ur Rehman A, Wilson A, Perreau F, Boulay C, Vass I, Krieger-Liszkay A, Kirilovsky D (2014) The Cyanobacterial Photoactive Orange Carotenoid Protein Is an Excellent Singlet Oxygen Quencher. Plant Cell 26: 1781-1791
Wilson A, Ajlani G, Verbavatz JM, Vass I, Kerfeld CA, Kirilovsky D (2006) A soluble carotenoid protein involved in phycobilisome-related energy dissipation in cyanobacteria. Plant Cell 18: 992-1007
Wilson A, Punginelli C, Gall A, Bonetti C, Alexandre M, Routaboul JM, Kerfeld CA, van Grondelle R, Robert B, Kennis JT, Kirilovsky D (2008) A photoactive carotenoid protein acting as light intensity sensor. Proc. Natl. Acad. Sci. U. S. A. 105: 12075-12080
Yasuhiro N, Eiji Y, Wataru (2007), Quenching Activities of Common Hydrophilic and Lipophilic Antioxidants against Singlet Oxygen Using Chemiluminescence Detection System. Carotenoid Science, Vol.11, 2007, 16-20
Zagalsky PF, Mummery RS, Eliopoulos EE, Findlay JBC (1990) The Quaternary Structure of the Lobster Carapace Carotenoprotein, Crustacyanin - Studies Using Cross-Linking Agents. Comparative Biochemistry and Physiology B-Biochemistry & Molecular Biology 97: 837-848.

## Claims

1. A method for producing a carotenoid-protein complex *in vivo* comprising the steps of :
a) transforming of prokaryote cells with at least one plasmid containing genes involved in carotenoid synthesis and with a plasmid containing an apo-carotenoprotein gene, wherein said plasmids contain different replication origins and different selective pressure;
b) culturing of transformed prokaryote cells in step a) in conditions allowing sequential gene expression,
c) isolating and purifying the carotenoid- protein complex expressed by the prokaryote cells.

2. The method according to claim 1 wherein the at least one plasmid containing genes involved in carotenoid synthesis in step a) is a plasmid containing the genes involved in ß-carotene synthesis.

3. The method according to claim 2) wherein the transforming of step a) further comprising a second plasmid containing the genes encoding a ß-carotene ketolase and/or the genes encoding a ß-carotene hydrolase.

4. The method according to any one of claims 1 to 3, where in the plasmid containing the genes involved in ß-carotene synthesis further contains the genes encoding a ß-carotene ketolase and/or *the* genes encoding ß-carotene hydrolase under the control of an inducible promoter, preferably *ara* promoter.

5. The method according to any one of claims 1 to 4, wherein step b) of culturing the prokaryote cells comprises the following steps:
b1) expressing the genes encoding ß-carotene without specific induction;
b2) inducing the gene expression of ß-carotene ketolase and/or ß-carotene hydrolase at temperature ranges of 33 to 40°C, preferably of 35 to 38 and more preferably about 37°C;
b3) inducing the gene expression of apo-carotenoprotein at temperature ranges of 20 to 30°C, preferably of 22 to 28°C and more preferably of 24 to 26°C.

6. The method of any one of claims 1 to 5 wherein apo-carotenoprotein gene is modified by introducing or deleting 9 to 45 nucleotides preferably 24 to 30 nucleotides just after the firs ATG of 5' end or just before the stop codon of said gene.

7. The method according to any one of claims 1 to 6, wherein the carotenoid genes are obtained from any bacteria, any algae or any plant containing carotenoids, preferably from cyanobacteria and non photosynthetic eubacteria and the said apo-carotenoprotein gene is isolated from any organism, preferably from cyanobacteria.

8. The method according to any claims 1 to 7, wherein the prokaryote cells are selected in the group of non-photosynthetic prokaryotic cells preferably comprising *E. coli* or *Lactococcus lactis.*

9. A carotenoid-protein complex obtained by the method according to any one of claims 1 to 8.

10. The carotenoid-protein complex according to claim 9 which is a protein selected in the group of soluble proteins, preferably comprising an orange carotenoid protein (OCP), a red carotenoid protein (RCP), AstaP, crustacyanin, glutathione s-transferasa like protein (GSTP1).

11. A modified gene encoding apo- OCP or apo-RCP **characterized in that** it is modified by introducing 9 to 45 nucleotides, preferably 24 to 30 nucleotides just after the firs ATG of 5' end and/or just before the stop codon of said gene.

12. A modified gene encoding apo- OCP according to claim 11 wherein the modifications just after the firs ATG of 5' end are selected from the group of sequences comprising: SEQ ID NOs: 8, 9, 10, 11, 12, 14, 15, 16, 17 and the modification just before the stop codon corresponds to SEQ ID NO: 18.

13. A modified gene encoding apo-RCP according to claim 11 wherein the modifications just after the firs ATG of 5' end are selected in the group of sequences comprising: SEQ ID NOs: 11, 13, and the modification just before the stop codon corresponds to SEQ ID NO: 18.

14. A vector, a plasmid or a host cell comprising a modified apo-carotenoprotein gene according to any one of claims 11 to 13.

15. The modified OCP or RCP encoded by the modified apo-protein gene according to any one of claim 11 to 13.
